(19) Europäisches Patentamt
European Patent Office
Office européen des brevets

(11) Veröffentlichungsnummer : **0 445 069 A1**

(12) # EUROPÄISCHE PATENTANMELDUNG

(21) Anmeldenummer : **91810089.2**

(22) Anmeldetag : **07.02.91**

(51) Int. Cl.[5] : **A01N 43/40**, C07D 213/53, C07D 405/12, // (C07D405/12, 309:00, 213:00)

(30) Priorität : **16.02.90 CH 511/90**

(43) Veröffentlichungstag der Anmeldung : **04.09.91 Patentblatt 91/36**

(84) Benannte Vertragsstaaten : **AT BE CH DE DK ES FR GB GR IT LI LU NL SE**

(71) Anmelder : **DR. R. MAAG AG**
**CH-8157 Dielsdorf (CH)**

(72) Erfinder : **Hostettler, Bernhard, Dr.**
**Im Obstgarten 7**
**CH-8044 Zürich (CH)**
Erfinder : **Wälder, Ludwig Rudolf, Dr.**
**Buchenrain 2**
**W-7893 Jestetten-Altenburg (DE)**

(74) Vertreter : **Roth, Bernhard M. et al**
**Patentabteilung CIBA-Geigy AG, Postfach**
**CH-4002 Basel (CH)**

(54) **Heterocyclische Verbindungen.**

(57) Es werden Unkrautbekämpfungmittel beschrieben, die durch einen Gehalt an herbizid wirksamen Verbindungen der Formel

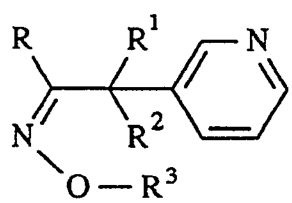

I

worin R, $R^1$, $R^2$ und $R^3$ die in der Beschreibung angegebenen Bedeutungen besitzen, deren N-Oxiden sowie den Säureadditionssalzen der Verbindungen I oder ihrer N-Oxide, gekennzeichnet sind, sowie die Verwendung dieser Substanzen und der Mittel beschrieben. Ebenfalls beschrieben werden gewisse neue Verbindungen der Formel I, N-Oxide und Säureadditionssalze und deren Herstellung.

EP 0 445 069 A1

## HETEROCYCLISCHE VERBINDUNGEN

Die vorliegende Erfindung betrifft Unkrautbekämpfungsmittel, die durch einen Gehalt an herbizid wirksamen heterocyclischen Verbindungen gekennzeichnet sind. Diese Verbindungen sind 2-(3-Pyridyl)-acetophenonoxime der allgemeinen Formel

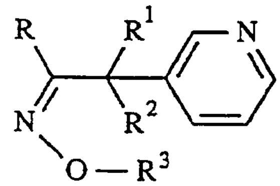

I

worin
R eine gegebenenfalls substituierte Phenylgruppe der Formel

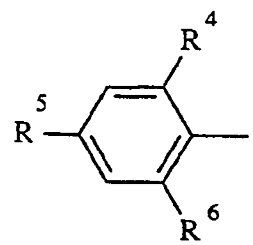

(a)

$R^1$ und $R^2$ unabhängig voneinander Wasserstoff, $C_n$-Alkyl, $C_n$-Alkenyl oder $C_n$-Alkinyl, wobei n eine Zahl von 1-4 bedeutet und $R^1$ oder $R^2$ für Wasserstoff stehen, wenn n grösser als 2 ist,
$R^3$ Wasserstoff; $C_{1-10}$-Alkyl; $C_{3-10}$-Alkoxyalkyl oder $C_{3-10}$-Alkylthioalkyl mit je einer Alkylenkette von mindestens 2 Kohlenstoffatomen; Aryl; Aryl-$C_{1-3}$-alkyl; $C_{3-6}$-Alkenyl; $C_{3-6}$-Alkinyl; oder eine Gruppe

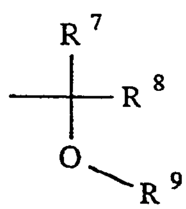

(b)

$R^4$ Wasserstoff oder Fluor,
$R^5$ und $R^6$ unabhängig voneinander Wasserstoff, Halogen, $C_{1-4}$-Alkyl oder Trifluormethyl,
$R^7$ und $R^8$ unabhängig voneinander Wasserstoff, $C_{1-6}$-Alkyl, $C_{3-6}$-Alkenyl oder $C_{3-6}$-Alkinyl
oder
$R^7$ und $R^8$ zusammen gegebenenfalls mit $C_{1-3}$-Alkyl mono- oder mehrfach substituiertes Tetra- oder Pentamethylen,
und
$R^9$ $C_{1-10}$-Alkyl; $C_{3-6}$-(Alkoxy-alkyl) oder $C_{3-6}$-(Alkylthio-alkyl) mit je einer Alkylenkette von mindestens zwei Kohlenstoffatomen; Aryl-$C_{1-3}$-alkyl; gegebenenfalls mit $C_{1-3}$-Alkyl mono- oder mehrfach substituiertes $C_{3-6}$-Cycloalkyl; $C_{3-10}$-Alkenyl; $C_{3-10}$-Alkinyl; Aryl,
oder
$R^7$ Wasserstoff
und
$R^8$ und $R^9$ zusammen gegebenenfalls mit $C_{1-3}$-Alkyl mono- oder mehrfach substituiertes Tetramethylen,
wobei die Gruppe (b) die schon oben in $R^3$ definierte $C_{3-10}$-Alkoxyalkylgruppe nicht umfassen soll,
deren N-Oxide sowie die Säureadditionssalze der Verbindungen I und der entsprechenden N-Oxide bedeuten.

In der obigen Formel I umfasst "Halogen" jeweils Fluor, Chlor, Brom und Jod. Die Alkyl-, Alkenyl- und Alkinylreste können geradkettig oder verzweigt sein, wobei dies auch für den Alkyl-, Alkenyl- bzw. Alkinylteil der Arylalkyl-, und allfällige weitere Alkyl-, Alkenyl- bzw. Alkinyl-enthaltende Gruppen gilt. Unter "Aryl" ist insbesondere Phenyl zu verstehen. Solche aromatischen Gruppen können einen oder mehrere, gleiche oder verschiedene Substituenten aufweisen, wie beispielsweise Halogen, Hydroxy,$C_{1-3}$-Alkyl (insbesondere Methyl), $C_{1-3}$-Alkoxy (insbesondere Methoxy), Trifluormethyl, Nitro und/oder Cyano. Dies gilt auch für den Arylteil einer Aryl-enthaltenden Gruppe,wie Aryl-alkyl.

Im Falle der $C_{3-10}$-Alkylthioalkyl-, $C_{3-10}$-Alkoxyalkyl, bzw. der $C_{3-8}$-(Alkyl-thioalkyl)-oder $C_{3-8}$-(Alkoxy-alkyl)-Gruppen bedeutet der in diesem Zusammenhang verwendete Ausdruck "Alkylenkette" die Kette der Kohlenstoffatome, die sich zwischen dem Schwefel- bzw. Sauerstoffatom der jeweiligen Gruppe und der freien Bindung erstreckt. Beispiele solcher Gruppen sind 2-Methylthio-ethyl, 3-Methoxy-propyl und 2-Methyl-thio-propyl (die erst- und drittgenannten Gruppen weisen eine Alkylenkette von 2 Kohlenstoffatomen auf, die zweitgenannte eine von 3 Kohlenstoffatomen).

In den Verbindungen der Formel I können asymmetrische Kohlenstoffatome vorhanden sein, so dass die Verbindungen als Enantiomeren- oder Diastereomerengemische vorliegen können. Durch das Vorliegen der C=N-Doppelbindung und gegebenenfalls auch einer aliphatischen C=C-Doppelbindung tritt geometrische Isomerie auf. Die Formel I soll demnach all diese möglichen isomeren Formen sowie die Isomerengemische umfassen.

Als Säureadditionssalze der Verbindungen I kommen physiologisch verträgliche Salze in Frage. Hierzu gehören vorzugsweise Salze der Verbindungen I mit anorganischen oder organischen Säuren wie Salzsäure, Salpetersäure, Phosphorsäure, mono- und bifunktionellen Carbonsäuren und Hydroxycarbonsäuren, z.B. Essigsäure, Maleinsäure, Bernsteinsäure Fumarsäure, Weinsäure, Zitronensäure, Salicylsäure, Sorbinsäure und Milchsäure, und Sulfonsäuren, z.B. 1,5-Naphthalindisulfonsäure. Im Falle der N-Oxide, d.h. der Verbindungen der Formel I, worin die 3-Pyridylgruppe ein Sauerstoffatom trägt (N→O), kommen als Säureadditionssalze insbesondere physiologisch verträgliche Salze mit starken Säuren in Frage, wie anorganische Säuren, z.B. Salzsäure, Salpetersäure und Phosphorsäure, und Sulfonsäuren, z.B. 1,5-Naphthalindisulfonsäure.

Unabhängig voneinander bedeuten R vorzugsweise 2,4- oder 2,6-Dihalogenphenyl, insbesondere 2-Chlor-4-fluor-phenyl, 4-Chlor-2-fluorphenyl, 2,4-Dichlorphenyl, 2-Chlor-6-fluorphenyl oder 2,6-Difluorphenyl, aus denen 2-Chlor-4-fluorphenyl und 2,6-Difluorphenyl sowie 2-Chlor-6-fluorphenyl als besonders bevorzugte Gruppen (a) gelten; $R^1$oder $R^2$ vorzugsweise Wasserstoff und $R^2$ bzw. $R^1$ Wasserstoff oder Alkyl, insbesondere Wasserstoff oder Methyl, und $R^3$ vorzugsweise tert.Butyl oder eine Gruppe (b), in der $R^7$, $R^8$ und $R^9$ unabhängig voneinander $C_{1-10}$-Alkyl, insbesondere alle drei Methyl oder $R^7$ Ethyl, $R^8$ n-Propyl und $R^9$ Methyl bedeuten.

Besonders bevorzugte Verbindungen der Formel I sind:
2',4'-Dichlor-2-(3-pyridyl)-acetophenon-O-(tert.butyl)-oxim,
2',4'-Dichlor-2-(3-pyridyl)-acetophenon-O-(1-methoxy-1-methylethyl)oxim,
2'-Chlor-4'-fluor-2-(3-pyridyl)-acetophenon-O-(1-methoxy-1-methylethyl)oxim
2',6'-Difluor-2-(3-pyridyl)-acetophenon-O-(1-methoxy-1-methylethyl)oxim
4'-Chlor-2'-fluor-2-(3-pyridyl)-acetophenon-O-(1-methoxy-1-methylethyl)oxim und
2'-Chlor-4'-fluor-2-(3-pyridyl)-acetophenon-O-(1-ethyl-1-methoxy-n-butyl)oxim.

Weitere Vertreter von Verbindungen I sind:
2',4'-Dichlor-2-methyl-2-(3-pyridyl)-propiophenon-O-(1-methoxy-1-methylethyl)oxim,
2'-Chlor-4'-fluor-2-methyl-2-(3-pyridyl)-propiophenon-O-(1-methoxy-1methyl-ethyl)oxim,
2',4'-Dichlor-2-(3-pyridyl)-acetophenon-O-(1-ethoxyethyl)oxim,
2'-Chlor-4'-fluor-2-(3-pyridyl)-acetophenon-O-(1-ethoxyethyl)oxim,
2',4'-Dichlor-2-(3-pyridyl)-acetophenon-O-(1-methoxyethyl)oxim,
2'-Chlor-4'-fluor-2-(3-pyridyl)-acetophenon-O-(1-methoxyethyl)oxim,
2',4'-Dichlor-2-(3-pyridyl)-acetophenon-O-(1-methoxypropyl)oxim,
2'-Chlor-4'-fluor-2-(3-pyridyl)-acetophenon-O-(1-methoxypropyl)oxim,
2',4'-Dichlor-2-(3-pyridyl)-propiophenon-O-methoxymethyloxim,
2'-Chlor-4'-fluor-2-(3-pyridyl)-propiophenon-O-methoxymethyloxim,
2',4'-Dichlor-2-(3-pyridyl)-acetophenon-O-(1-methoxycyclohexyl)oxim,
2',4'-Dichlor-2-(3-pyridyl)-acetophenon-O-(2-tetrahydropyranyl)oxim und
2'-Chlor-4'-fluor-2-(3-pyridyl)-acetophenon-O-(2-tetrahydropyranyl)oxim.

Im allgemeinen zeichnen sich die (E)-Isomeren als wirksamere Herbizide als die (E/Z)-Isomerengemische aus, die ihrerseits eine höhere herbizide Aktivität aufweisen als die (Z)-Isomeren.

Die Verbindungen der Formel I und deren N-Oxide und Säureadditionssalze sind teilweise neu und teilweise bekannt, siehe z.B. europäische Patentanmeldung EP-A-0 049 854 und deutsche Offenlegungsschriften DE-OS 3.309.466 und 3.310.148, welche die bekannten Verbindungen und deren Eigenschaften als Fungizide beinhalten. Die neuen Verbindungen lassen sich demzufolge wie folgt definieren:

Verbindungen der allgemeinen Formel

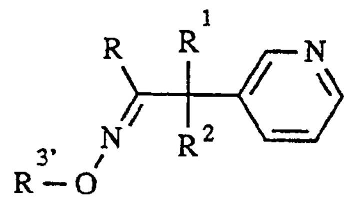

I'

worin

R, $R^1$ und $R^2$ die unter Formel I angegebenen Bedeutungen besitzen und

$R^{3'}$ $C_{3-10}$-(Alkylthio-alkyl) mit einer Alkylenkette von mindestens zwei Kohlenstoffatomen, oder eine Gruppe (b), wie dies oben definiert ist, bedeutet,

deren N-Oxide sowie die Säureadditionssalze der Verbindungen I' und ihrer N-Oxide.

Diese neuen Verbindungen der Formel I', sowie deren N-Oxide und Säureadditionssalze und das Verfahren zur Herstellung dieser neuen Verbindungen sind ebenfalls Gegenstand der vorliegenden Erfindung.

Die grösstenteils bekannten Verbindungen der Formel I, d.h. diejenigen Verbindungen der Formel I, in denen R, $R^1$ und $R^2$ die oben angegebenen Bedeutungen besitzen und $R^3$ Wasserstoff; $C_{1-10}$-Alkyl; $C_{3-10}$-Alkoxyalkyl; Aryl; Aryl-$C_{1-3}$-alkyl; $C_{3-6}$-Alkenyl; $C_{3-6}$-Alkinyl bedeuten, sowie deren N-Oxide und die Säureadditionssalze dieser Verbindungen können nach den in der europäischen Patentpublikation EP-A-0 049 854 und in den deutschen Offenlegungsschriften 3.309.466 und 3.310.148 beschriebenen Methoden oder auf analoge Weise hergestellt werden.

Das erfindungsgemässe Verfahren zur Herstellung der neuen Verbindungen der Formel I', N-Oxide sowie Säureadditionssalze ist dadurch gekennzeichnet, dass man

a) zwecks Herstellung derjenigen Verbindungen der Formel I', in denen $R^1$ und $R^2$ Wasserstoff bedeuten, und $R^{3'}$ verschieden von einer Gruppe (b) ist, ein Enamin der allgemeinen Formel

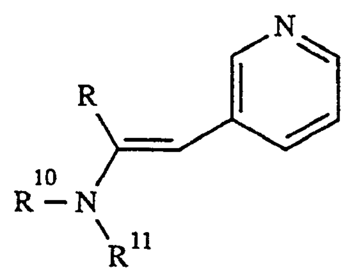

II

worin

R die oben angegebene Bedeutung besitzt

und

$R^{10}$ und $R^{11}$ unabhängig voneinander $C_{1-4}$-Alkyl, insbesondere Methyl oder Ethyl, bedeuten,

mit einem O-substituierten Hydroxylamin der allgemeinen Formel

$$R^{3''}-O-NH_2 \qquad III$$

worin

$R^{3''}$ $C_{1-10}$-Alkyl; $C_{3-10}$-Alkoxyalkyl oder $C_{3-10}$-Alkylthioalkyl mit je einer Alkylenkette von midestens 2 Kohlenstoffatomen; Aryl; Aryl-$C_{1-3}$-alkyl; $C_{3-6}$-Alkenyl; $C_{3-6}$-Alkinyl bedeutet,

oder mit einem anorganischen Säureadditionssalz davon umsetzt,

b) zwecks Herstellung derjenigen Verbindungen der Formel I' und von deren N-Oxiden, in denen $R^{3'}$ verschieden von einer Gruppe (b) ist, ein Acetophenonderivat oder dessen N-Oxid der allgemeinen Formel

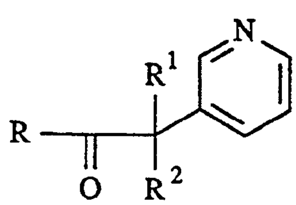

IV

worin R, $R^1$ und $R^2$ die oben angegebenen Bedeutungen besitzen, mit einem O-substituierten Hydroxylamin der oben angegebenen allgemeinen Formel III oder mit einem anorganischen Säureadditionssalz davon oder mit Hydroxylamin-hydrochlorid umsetzt,

c) zwecks Herstellung derjenigen Verbindungen der Formel I', in denen $R^{3'}$ verschieden von einer Gruppe (b) ist, und von deren N-Oxiden, ein Oxim oder dessen N-Oxid der allgemeinen Formel

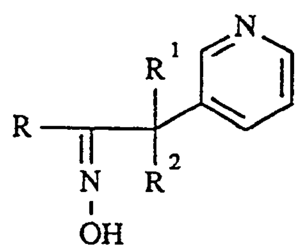

V

worin R, $R^1$ und $R^2$ die oben angegebenen Bedeutungen haben, mit einer Verbidung der allgemeinen Formel

$R^{3'''}$ — X     VI

worin

$R^{3'''}$ $C_{3-6}$-(Alkylthio-alkyl) oder $C_{3-6}$-(Alkoxy-alkyl) mit einer Alkylenkette von mindestens zwei Kohlenstoffatomen; Aryl-$C_{1-3}$-alkyl; gegebenenfalls mit $C_{1-3}$-Alkyl mono- oder mehrfach substituiertes $C_{3-6}$-Cycloalkyl; $C_{3-10}$-Alkenyl; $C_{3-10}$-Alkinyl;

und

X eine Abgangsgruppe, z.B. Chlor, Brom, Jod, Mesyloxy, Tosyloxy oder einen Alkylsulfatrest, bedeuten, umsetzt,

d) zwecks Herstellung derjenigen Verbindungen der Formel I' und deren N-Oxiden, in denen $R^{3'}$ eine Gruppe (b) bedeutet, in der aber $R^8$ und $R^9$ miteinander nicht verknüpft sind, ein Oxim der oben angegebenen allgemeinen Formel V oder dessen N-Oxid mit einem Diether der allgemeinen Formel

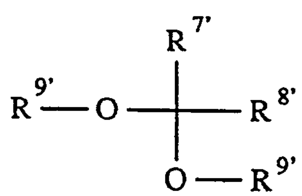

VII

worin $R^{7'}$, $R^{8'}$ und $R^{9'}$ die oben angegebenen einzelnen Bedeutungen von $R^7$, $R^8$ bzw. $R^9$ besitzen und zudem $R^{7'}$ und $R^{8'}$ zusammen gegebenenfalls mit $C_{1-3}$-Alkyl mono- oder mehrfach substituiertes Tetra- oder Pentamethylen bedeuten können, umsetzt, oder

e) zwecks Herstellung derjenigen Verbindungen der Formel I' und deren N-Oxiden, in denen $R^{3'}$ eine Gruppe (b) bedeutet, in der $R^7$ Wasserstoff und $R^8$ und $R^9$ zusammen gegebenenfalls mit $C_{1-3}$-Alkyl mono- oder mehrfach substituiertes Tetramethylen bedeuten, ein Oxim der oben angegebenen allgemeinen Formel V oder dessen N-Oxid mit einer Verbindung der allgemeinen Formel

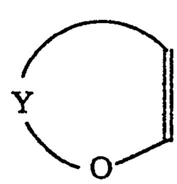

VIII

worin Y gegebenenfalls mit $C_{1-3}$-Alkyl mono- oder mehrfach substituiertes Trimethylen bedeutet, umsetzt.

Die Umsetzung gemäss der Verfahrensvariante a) erfolgt zweckmässigerweise in einem stark sauren, wässrigen Verdünnungsmittel, wie Schwefelsäure, bei Temperaturen zwischen 0°C und 100°C, vorzugsweise bei 60-75°C. Falls das Hydroxylamin als Säureadditionssalz davon eingesetzt wird, ist dies geeigneterweise

ein Salz mit einer anorganischen Säure, vorzugsweise das Hydrochlorid.

Die Verfahrensvariante b) wird zweckmässigerweise dadurch durchgeführt, indem man das Acetophenonderivat der Formel IV oder dessen N-Oxid mit dem Hydroxylamin der Formel III, oder mit einem Säureadditionssalz davon, vorzugsweise dem Hydrochlorid oder Hydrosulfat, in einem organischen Lösungsmittel, z.B. einem Alkohol, wie Methanol oder Ethanol, einem Dialkylamid, wie Dimethylformamid, oder einem tertiären Amin, wie Pyridin, umsetzt. Man arbeitet vorzugsweise in einem Temperaturbereich zwischen Raumtemperatur und der Rückflusstemperatur des Reaktionsgemisches. Da das Hydroxylamin III vorzugsweise in Form eines Säureadditionssalzes, z.B. des Hydrochlorids oder Hydrosulfats, eingesetzt wird, setzt man dem Reaktionsgemisch zweckmässigerweise eine Base, wie Natrium- oder Kaliumcarbonat, Triethylamin oder Pyridin zu.

Unter Verwendung einer Verbindung der Formel VI als Reagens kann die Verfahrensvariante c) durchgeführt werden, indem man das Oxim der Formel V oder dessen N-Oxid mit der Verbindung der Formel VI, zweckmässigerweise in Gegenwart einer Base, in einem organischen Lösungsmittel und in einem Temperaturbereich zwischen 0°C und der Rückflusstemperatur des Reaktionsgemisches umsetzt. Das Lösungsmittel kann protisch oder nicht protisch sein. Im Fall von protischen Lösungsmitteln, wie Alkohol, insbesondere Methanol oder Ethanol, ist die verwendete Base vorzugsweise ein Alkalimetallhydroxid, z.B. Natrium- oder Kaliumhydroxid, oder ein Alkalimetallalkoholat. Bei Verwendung von nichtprotischen Lösungsmitteln, wie aliphatischen oder cyclischen Ethern, insbesondere 1,2-Dimethoxyethan bzw. Tetrahydrofuran, und Dialkylamiden, insbesondere Dimethylformamid, ist die Base vorzugsweise ein Alkalimetallhydrid, z.B. Natriumhydrid. In einer bevorzugten Ausführungsform dieses Verfahrens werden Natriumhydrid als Base und ein aliphatischer oder cyclischer Ether, insbesondereTetrahydrofuran oder Dimethoxyethan, oder ein Dialkylamid, insbesondere Dimethylformamid, als Lösungsmittel verwendet.

Die Verfahrensvariante d) wird zweckmässigerweise unter Verwendung des Diethers der Formel VII sowohl als Reagens als auch als Lösungsmittel durchgeführt. Die Umsetzung erfolgt geeigneterweise bei Temperaturen zwischen Raumtemperatur und der Rückflusstemperatur des Reaktionsgemisches und in Gegenwart einer katalytischen Menge einer aprotischen Säure, wie beispielsweise Methansulfonsäure, p-Toluolsulfonsäure oder eines stark sauren Kunstharzes, z.B. Amberlyst® A15. Als Alternative kann man das Oxim V oder dessen N-Oxid mit 1 bis 5 Aequivalenten, vorzugsweise ca. 2 Aequivalenten des Diethers VII, in Gegenwart eines polaren Cosolvens, z.B. eines Xylols, 1,3-Dimethyl-3,4,5,6-tetrahydro-2(1H)-pyrimidinon oder insbesondere Dioxan, bei Temperaturen zwischen 0°C und der Rückflusstemperatur des Reaktionsgemisches, vorzugsweise bei ca. 90-100°C, reagieren lassen.

Die Umsetzung gemäss der Verfahrensvariante e) erfolgt zweckmässigerweise unter Verwendung der Verbindung der formel VIII sowohl als Reagens als auch Lösungsmittel, bei Temperaturen zwischen O°C und 50°C, insbesondere bei Raumtemperatur, und in Gegenwart einer katalytischen Menge einer Mineralsäure, z.B. Salzsäure. Näheres zu diesem Reaktionstyp findet sich in J.A.C.S. 70, 4187 (1948).

Die fakultative N-Oxidation eines Endproduktes der Formel I, das nicht bereits in Form des N-Oxids vorliegt, kann zweckmässigerweise durchgeführt werden, indem man die Verbindung der Formel I' mittels einer Persäure in Gegenwart eines inerten Verdünnungsmittels N-oxidiert. Verbindungen der Formel I', in denen $R^{3'}$ eine Gruppe (b) bedeutet, lassen sich nicht N-oxidieren (in solchen Fällen können die N-Oxide bereits in der Verfahrensvariante d) oder e), also unter Verwendung eines N-Oxids des Oxims der Formel V, hergestellt werden).

Als Persäuren kommen vorzugsweise Peressigsäure, Perbenzoesäure und m-Chlorperbenzoesäure in Frage, wobei bevorzugt in einem halogenierten Kohlenwasserstoff, z.B. Methylenchlorid oder Chloroform, als Verdünnungsmittel gearbeitet wird. Die N-Oxidation mit einer Persäure erfolgt vorzugsweise in einem Temperaturbereich zwischen 0°C und der Rückflusstemperatur des Reaktionsgemisches, insbesondere zwischen 0°C und der Raumtemperatur. Eine besonders bevorzugte Ausführungsform dieses Verfahrens besteht darin, dass man die N-Oxidation mit m-Chlorperbenzoesäure in Chloroform in einem Temperaturbereich zwischen 0°C und der Raumtemperatur durchführt.

Zur Herstellung von Säureadditionssalzen können erwünschtenfalls die Verbindungen der Formel I' bzw. deren N-Oxide mit anorganischen oder organischen Säuren, wie z.B. Salzsäure, Salpetersäure, Phosphorsäure, mono- und bifunktionellen Carbonsäuren und Hydroxycarbonsäuren oder Sulfonsäuren, in an sich bekannter Weise umgesetzt werden. In der Regel jedoch lassen sich diejenigen Verbindungen der Formel I, in denen $R^{3'}$ eine Gruppe (b) bedeutet, nicht in ihre Säureadditionssalze umwandeln, da sie besonders säureempfindlich sind.

Die Isolierung und Reinigung der so hergestellten Verbindungen der Formel I', N-Oxide bzw. Säureadditionssalze erfolgen nach an sich bekannten Methoden.

Die als Ausgangsmaterialien verwendbaren Verbindungen der Formeln II-IV, VII-VIII und N-Oxide der Verbindungen IV und V, anorganischen Säureadditionssalze der Verbindungen III sind entweder bekannt, oder können nach an sich bekannten Methoden hergestellt werden. Bei den als Ausgangsmaterialien verwendbaren Verbindungen der Formel V handelt es sich um eine Untergruppe von grösstenteils bekannten Verbindungen

der Formel I (siehe beispielsweise EP-A-0 049 854 und DE-OS 3.310.148).

Die Verbindungen der Formel I, N-Oxide und Säureadditionssalze (im folgenden insgesamt als "Wirkstoffe" bezeichnet) besitzen herbizide Eigenschaften und eignen sich zur Bekämpfung von Unkräutern, einschliesslich Ungräser, u.a. Agropyron repens. Alopecurus myosuroides, Digitaria sanguinalis, Bromus inermis, Echinochloa crus-galli, Poa annua, Sorghum halepense, Abutilon theophrasti, Amaranthus retroflexus, Chrysanthemum segetum, Sinapis arbensis und Stellaria media, in diversen Nutzpflanzenkulturen, u.a. Kulturen von Oryza sativa (Reis), Triticum aestivum (Weizen), Zea mays (Mais), Beta vulgaris (Zuckerrübe), Brassica napus (Raps), Glycine max (Soja) und Gossypium hirsutum (Baumwolle). Zudem sind die Wirkstoffe sowohl Vorauflauf- als auch Nachauflauf-Herbizide. Bei einigen Vertretern der Wirkstoffe hat sich eine sehr gute Selektivität gezeigt, insbesondere bei der Bekämpfung von Unkräutern in Wasserreiskulturen. Dort werden von verschiedenen Reis-Varietäten, wie beispielsweise Koshihikari und Labelle, Konzentrationen der Wirkstoffe toleriert, die erheblich über den Dosierungen liegen, durch welche die wichtigsten Reis-Unkräuter, einschliesslich Ungräser, kontrolliert werden. Beispiele solcher in Reiskulturen befindlichen Unkräuter sind Echinochloa crus-galli, Echinochloa caudata, Monochoria vaginalis, Lindernia procumbens, Sagittaria pygmaea, Scirpus juncoides und Cyperus serotinus.

In der Praxis genügt üblicherweise eine Konzentration von 1 g bis 3 kg Wirkstoff/ha, vorzugsweise 10 g bis 1 kg Wirkstoff/ha, um den gewünschten herbiziden Effekt zu erzielen. Um den gewünschten herbiziden Effekt bei optimaler Nutzpflanzen-Verträglichkeit zu erzielen, ist der Bereich von 10 bis 300 g/ha in der Vorauflaufbehandlung und von 300 bis 3000 g/ha in der Nachauflaufbehandlung besonders günstig.

Das erfindungsgemässe Unkrautbekämpfungsmittel ist dadurch gekennzeichnet, dass es eine wirksame Menge mindestens einer Verbindung der Formel I, wie oben definiert, eines N-Oxids davon oder eines Säureadditionssalzes einer solchen Verbindung I bzw. eines solchen N-Oxids, sowie Formulierungshilfsstoffe enthält. Das Mittel enthält zweckmässigerweise zumindest einen der folgenden Formulierungshilfsstoffe aus der Gruppe: feste Trägerstoffe; Lösungs- bzw. Dispersionsmittel; Tenside (Netzmittel und Emulgatoren); Dispergiermittel (ohne Tensidwirkung); und Stabilisatoren. Unter Verwendung solcher und anderer Hilfsstoffe können diese Verbindungen, also die herbiziden Wirkstoffe, in die üblichen Formulierungen übergeführt werden, wie Stäube, Pulver, Granulate, Lösungen, Emulsionen, Suspensionen, emulgierbare Konzentrate, Pasten und dergleichen.

Die Verbindungen der Formel I und deren N-Oxide sind im allgemeinen wasserunlöslich, die Säureadditionssalze hingegen wasserlöslich, und können nach den für wasserunlösliche bzw. wasserlösliche Verbindungen üblichen Methoden unter Verwendung der diesbezüglichen Formulierungshilfsstoffe konfektioniert werden. Die Herstellung der Mittel kann in an sich bekannter Weise durchgeführt werden, z.B. durch Vermischen des jeweiligen Wirkstoffes mit festen Trägerstoffen, durch Auflösen oder Suspendieren in geeigneten Lösungs- bzw. Dispersionsmitteln, eventuell unter Verwendung von Tensiden als Neztmitteln oder Emulgatoren und/oder von Dispergiermitteln, durch Verdünnen bereits vorbereiteter emulgierbarer Konzentrate mit Lösungs- bzw. Dispersionsmitteln usw.

Als feste Trägerstoffe kommen im wesentlichen in Frage: natürliche Mineralstoffe, wie Kreide, Dolomit, Kalkstein, Tonerden und Kieselsäure und deren Salze (beispielsweise Kieselgur, Kaolin, Bentonit, Talkum, Attapulgit und Montmorillonit); synthetische Mineralstoffe, wie hochdisperse Kieselsäure, Aluminiumoxid und Silikate; organische Stoffe, wie Cellulose, Stärke, Harnstoff und Kunstharze; und Düngemittel, wie Phosphate und Nitrate, wobei solche Trägerstoffe z.B. als Pulver oder als Granulate vorliegen können.

Als Lösungs- bzw. Dispersionsmittel kommen im wesentlichen in Frage: Aromaten und chlorierte aliphatische Kohlenwasserstoffe, wie Chlorbenzole, Chlorethylene und Methylenchlorid; aliphatischen Kohlenwasserstoffe, wie Cyclohexan und Paraffine, z.B. Erdölfraktionen; Alkohole, wie Butanol und Glykol, sowie deren Ether und Ester, Ketone, wie Aceton, Methylethylketon, Methylisobutylketon und Cyclohexanon; und stark polare Lösungs- bzw. Dispersionsmittel, wie Dimethylformamid, N-Methylpyrrolidon und Dimethylsulfoxid, wobei solche Lösungsmittel vorzugsweise Flammpunkte von mindestens 30°C und Siedepunkte von mindestens 50°C aufweisen, und Wasser. Unter den Lösungs- bzw. Dispersionsmitteln kommen auch in Frage sogenannte verflüssigte gasförmige Streckmittel oder Trägerstoffe, die solche Produkte sind, welche bei Raumtemperatur und unter Normaldruck gasförmig sind. Beispiele solcher Produkte sind insbesondere Aerosol-Treibgase, (wie Halogenkohlenwasserstoffe, z.B. Dichlordifluormethan oder andere neutrale Treibgase). Liegt das erfindungsgemässe Unkrautbekämpfungsmittel in Form einer Druckgaspackung vor, so wird zweckmässigerweise zusätzlich zum Treibgas ein Lösungsmittel verwendet.

Die Tenside (Netzmittel und Emulgatoren) können nicht-ionische Verbindungen sein, wie Kondensationsprodukte von Fettsäuren, Fettalkoholen oder fettsubstituierten Phenolen mit Ethylenoxid; Fettsäureester und -ether von Zuckern oder mehrwertigen Alkoholen; die Produkte, die aus Zuckern oder mehrwertigen Alkoholen durch Kondensation mit Ethylenoxid erhalten werden; Blockpolymere von Ethylenoxid und Propylenoxid; oder Alkyldimethylaminoxide.

Die Tenside können auch anionische Verbindungen sein, wie Seifen; Fettsulfatester, z.B. Dodecylnatriumsulfat, Octadecylnatriumsulfat und Cetylnatriumsulfat; Alkylsulfonate, Arylsulfonate und fettaromatische Sulfonate, wie Alkylbenzolsulfonate, z.B. Calciumdodecylbenzolsulfonat, und Butylnaphthalinsulfonate; und komplexere Fettsulfonate, z.B die Amidkondensationsprodukte von Oelsäure und N-Methyltaurin und das Natriumsulfonat von Dioctylsuccinat.

Die Tenside können schliesslich kationische Verbindungen sein, wie Alkyldimethylbenzylammoniumchloride, Dialkyldimethylammoniumchloride, Alkyltrimethylammoniumchloride und ethoxylierte quaternäre Ammoniumchloride.

Als Dispergiermittel (ohne Tensidwirkung) kommen im wesentlichen in Frage: Lignin, Natrium- und Ammoniumsalze von Ligninsulfonsäuren, Natriumsalze von Maleinsäureanhydrid-diisobutylen-Copolymeren, Natrium- und Ammoniumsalze von sulfonierten Polykondensationsprodukten aus Naphthalin und Formaldehyd, und Sulfitablaugen.

Als Dispergiermittel, die sich insbesondere als Verdickungs- bzw. Antiabsetzmittel eignen, können z.B. Methylcellulose, Carboxymethylcellulose, Hydroxyethylcellulose, Polyvinylalkohol, Alginate, Caseinate und Blutalbumin eingesetzt werden.

Beispiele von geeigneten Stabilisatoren sind säurebindende Mittel, z.B. Epichlorhydrin, Phenylglycidether und epoxidierte Soyaöle; Antioxidantien, z.B. Gallussäureester und Butylhydroxytoluol; UV-Absorber, z.B. substituierte Benzophenone, Diphenylacrylnitrilsäureester und Zimtsäureester; und Deaktivatoren, z.B. Salze der Ethylendiamintetraessigsäure und Polyglykole.

Die erfindungsgemässen Unkrautbekämpfungsmittel können zusätzlich zu den Wirkstoffen Synergisten und andere Wirkstoffe, z.B. Insektizide, Akarizide, Fungizide, Pflanzenwachstumsregulatoren und Düngemittel, enthalten. Solche Kombinationsmittel eignen sich zur Verstärkung der Aktivität bzw. zur Verbreiterung des Wirkungsspektrums.

Die erfindungsgemässen Unkrautbekämpfungsmittel enthalten im allgemeinen zwischen 0,001 und 99 Gewichtsprozent, vorzugsweise zwischen 0,1 und 90 Gewichtsprozent eines bzw. mehrerer Wirkstoffe der Formel I, 1-99 % eines festen oder flüssigen Zusatzstoffes wie z.B. feste Trägerstoffe, Lösungs- und Dispergiermittel sowie Stabilisatoren und 0 bis 25 % eines Tensids. Sie können z.B. in einer Form vorliegen, die sich für die Lagerung und den Transport eignet. In solchen Formulierungen, z.B. emulgierbaren Konzentraten, ist die Wirkstoffkonzentration normalerweise im höheren Bereich, vorzugsweise zwischen 5 und 80 Gewichtsprozent, insbesondere zwischen 10 und 50 Gewichtsprozent. Diese Formulierungen können dann, z.B. mit gleichen oder verschiedenen inerten Stoffen, bis zu Wirkstoffkonzentrationen verdünnt werden, die sich für den praktischen Gebrauch eignen, also vorzugsweise ca. 0,001 bis 10 Gewichtsprozent, insbesondere ca. 0,01 bis 5 Gewichtsprozent. Die Wirkstoffkonzentrationen können jedoch auch kleiner oder grösser sein.

Wie oben erwähnt, kann die Herstellung der erfindungsgemässen Unkrautbekämpfungsmittel in an sich bekannter Weise durchgeführt werden.

Zur Herstellung pulverförmiger Präparate kann der Wirkstoff bzw. können die Wirkstoffe mit festem Trägerstoff vermischt werden, z.B. durch Zusammenmahlen; oder man kann den festen Trägerstoff mit einer Lösung oder Suspension des Wirkstoffes imprägnieren und dann das Lösungs- bzw. Dispersionsmittel durch Abdunsten, Erhitzen oder Absaugen unter vermindertem Druck entfernen. Durch Zusatz von Tensiden bzw. Dispergiermitteln kann man solche pulverförmige Mittel mit Wasser leicht benetzbar machen, so dass sie in wässrige Suspensionen, die sich z.B. als Spritzmittel eignen, übergeführt werden können.

Der Wirkstoff kann auch mit einem Tensid und einem festen Trägerstoff zur Bildung eines netzbaren Pulvers vermischt werden, welches in Wasser dispergierbar ist, oder er kann mit einem festen vorgranulierten Trägerstoff zur Bildung eines granulatförmigen Produktes vermischt werden.

Wenn gewünscht, kann der Wirkstoff in einem mit Wasser nicht mischbaren Lösungsmittel, wie beispielsweise einem hochsiedenden Kohlenwasserstoff, gelöst werden, das zweckmässigerweise gelösten Emulgator enthält, so dass die Lösung bei Zugabe zu Wasser selbstemulgierend wirkt. Andernfalls kann der Wirkstoff mit einem Emulgator vermischt und das Gemisch dann mit Wasser auf die gewünschte konzentration verdünnt werden. Zudem kann der Wirkstoff in einem Lösungsmittel gelöst und danach mit einem Emulgator gemischt werden. Ein solches Gemisch kann ebenfalls mit Wasser auf die gewünschte Konzentration verdünnt werden. Auf diese Weise erhält man emulgierbare Konzentrate bzw. gebrauchsfertige Emulsionen.

Die Verwendung der erfindungsgemässen Unkrautbekämpfungsmittel, die einen weiteren Gegenstand der vorliegenden Erfindung bildet, kann nach üblichen Applikationsmethoden, wie Spritzen, Sprühen, Stäuben, Giessen oder Streuen, erfolgen. Das erfindungsgemässe Verfahren zur Bekämpfung von Unkräutern ist dadurch gekennzeichnet, dass man das gegen Unkräuter zu schützende Gut und/oder die Unkräuter mit einer Verbindung der Formel I, einem N-Oxid davon oder einem Säureadditionssalz der Verbindung I oder des N-Oxids bzw. mit einem erfindungsgemässen Unkrautbekämpfungsmittel behandelt.

Die nachfolgenden Beispiele dienen zur näheren Erläuterung der Erfindung.

I. Herstellung der Wirkstoffe:

Beispiel 1

Eine Lösung von 1,2 g O-Methylhydroxylamin-hydrochlorid in 2,4 ml destilliertem Wasser wird unter Rühren tropfenweise mit konzentrierter Schwefelsäure so langsam versetzt, dass die Reaktionstemperatur nicht über 60°C ansteigt. Danach tropft man 4,0 g 3-[β-(Diethylamino)-2-chlor-4-fluor-styryl]-pyridin so rasch hinzu, dass am Ende der Zugabe die Innentemperatur gerade 70°C beträgt. Das Gemisch wird anschliessend 5 Stunden bei 70°C gerührt, auf Raumtemperatur abgekühlt und auf Eiswasser gegossen. Die Wasserphase wird mit Natronlauge auf pH = 8,0 eingestellt und das Gemisch mit Diethylether extrahiert. Die organische Phase wird über wasserfreiem Magnesiumsulfat getrocknet und unter vermindertem Druck eingeengt. Man erhält das 2'-Chlor-4'-fluor-2-(3-pyridyl)-acetophenon-O-methyloxim als E/Z-Isomerengemisch in Form eines bräunlichen Oels. Säulenchromatographische Trennung an Kieselgel mit n-Hexan/Ethylacetat (4:1) ergibt zuerst das E-Isomere [$^1$H-NMR ($CDCl_3$, 200 MHz): 4,03 ppm (s, $C\underline{H}_3O$-N=)] und dann das Z-Isomere ($^1$H-NMR ($CDCl_3$, 200 MHz): 3,84 ppm (s, $C\underline{H}_3O$-N=)].

Beispiel 2

Eine Lösung von 4,65 g 2',4'-Dichlor-2-(3-pyridyl)-acetophenon in 25 ml Ethanol wird mit 2,70 g wasserfreiem Natriumcarbonat und 2,63 g O-Isobutylhydroxylamin-hydrochlorid versetzt und anschliessend unter Rühren auf Rückflusstemperatur erhitzt. Nach 4 Stunden wird das Reaktionsgemisch auf Eis gegossen und mit Ethylacetat extrahiert. Die organische Phase wird mit Wasser gewaschen, über wasserfreiem Magnesiumsulfat getrocknet und unter vermindertem Druck eingeengt. Man erhält das 2',4'-Dichlor-2-(3-pyridyl)-acetophenon-O-isobutyloxim als E/Z-Isomerengemisch in Form eines bräunlichen Oels. Bei der chromatographischen Trennung an Silicagel mit n-Hexan/Ethylacetat (7:3) wird zuerst das E-Isomere [$^1$H-NMR ($CDCl_3$, 200 MHz): 2,055 ppm (Heptett, $\underline{H}C(CH_3)_2$] und dann das Z-Isomere [$^1$H-NMR ($CDCl_3$, 200 MHz): 1,928 ppm (Heptett, $\underline{H}C(CH_3)_2$)] eluiert.

Beispiel 3

Eine Lösung von 3,6 g 2',4'-Dichlor-2-methyl-2-(3-pyridyl)-acetophenon in 15 ml Ethanol und 4 ml Wasser wird mit 1,4 g Hydroxylamin-hydrochlorid, 3 ml Pyridin und 90 mg 4-N,N-Dimethylamino-pyridin versetzt und auf Rückflusstemperatur erhitzt. Nach 16 Stunden wird das Reaktionsgemisch auf Eiswasser gegossen und mit Ethylacetat extrahiert. Die organische Phase wird über wasserfreiem Magnesiumsulfat getrocknet und unter vermindertem Druck eingeengt. Das kristalline Rohprodukt wird aus Methylenchlorid/n-Hexan umkristallisiert. Man erhält das 2',4'-Dichlor-2-methyl-2-(3-pyridyl)-acetophenonoxim als E/Z-Isomerengemisch in Form beiger Kristalle, Smp. 163-165°C.

Beispiel 4

Eine Lösung von 2',4'-Difluor-2-(3-pyridyl)-acetophenonoxim in 25 ml Dimethoxyethan wird portionenweise mit 0,50 g Natriumhydrid-Dispersion (55% in Oel) versetzt und 30 Minuten bei Raumtemperatur gerührt. Darauf gibt man 1,8 g Methyljodid zu und erhitzt das Gemisch auf Rückflusstemperatur. Nach 4 Stunden wird auf Raumtemperatur abgekühlt, auf Eis gegossen, mit Ethylacetat extrahiert, die organische Phase über wasserfreiem Magnesiumsulfat getrocknet und unter vermindertem Druck eingeengt. Nach säulenchromatographischer Trennung an Kieselgel erhält man zuerst das (E)-2',4'-Difluor-2-(3-pyridyl)-actophenon-O-methyloxim [$^1$H-NMR ($CDCl_3$, 200 MHz): 4,05 ppm (s, $C\underline{H}_3O$-N=)] und dann das (Z)-2',4'-Difluor-2-(3-pyridyl)-acetophenon-O-methyloxim [$^1$H-NMR ($CDCl_3$, 200 MHz): 3,91 ppm (s, $C\underline{H}_3O$-N=)].

Beispiel 5

Eine Lösung von 20,00 g 2'-Chlor-6'-fluor-2-(3-pyridyl)-acetophenonoxim wird in 170 ml 2,2-Dimethoxypropan gelöst und nach Zugabe von 0,5 g pulverisiertem Amberlyst® A15 unter Rühren auf Rückflusstemperatur erwärmt. Nach jeweils 12-14 Stunden werden ca. 30 ml aus dem Reaktionsgemisch abdestilliert und durch die gleiche Menge 2,2-Dimethoxypropan ersetzt. Nach 48 Stunden lässt sich dünnschichtchromatographisch kein Edukt mehr nachweisen. Das Reaktionsgemisch wird auf etwa 40°C abgekühlt und bei dieser Temperatur nach Filtration über Celite® bei 80 mbar am Rotationsverdampfer auf ca. ein Drittel des ursprünglichen Volumens eingeengt. Der Rückstand wird auf 150 ml Eiswasser gegossen und dreimal mit je 150 ml Diethylether

extrahiert. Die vereinigten Etherphasen werden einmal mit 200 ml Wasser gewaschen, über wasserfreiem Magnesiumsulfat getrocknet und eingedampft. Man erhält das 2'-Chlor-4'-fluor-2-(3-pyridyl)-acetophenon-O-(1-methoxy-1-methylethyl)oxim als E/Z-Isomerengemisch in Form eines gelben Oels. Nach Kieselgelchromatographie mit n-Hexan/Ethylacetat (3:2) fällt zuerst das E-Isomere [$^1$H-NMR ($CDCl_3$, 200 MHz): 4,08 ppm (s, $\underline{H_3}$C-O-C)] und dann das Z-Isomere [$^1$H-NMR ($CDCl_3$, 200 MHz): 3,97 ppm (s, $\underline{H_3}$C-O-C)] an.

Beispiel 6

Eine Lösung von 7,45 g 2',6'-Difluor-2-(3-pyridyl)-acetophenonoxim in 170 ml Xylol wird mit 8,00 g Diäthoxypropan vermischt und anschliessend unter Rühren auf 80°C erwärmt. Nachdem sich eine klare Lösung gebildet hat, gibt man 0,20 g pulverisierten Amberlyst$^R$ A15 hinzu und heizt das Ganze bis zur Rückflusstemperatur auf. Nach 6 Stunden wird das Reaktionsgemisch über Celite® klarfiltriert und das Filtrat bei 60°C am Wasserstrahlvakuum von überschüssigem Lösungsmittel befreit. Nach Trocknen am Hochvakuum erhält man das 2',6'-Difluor-2-(3-pyridyl)-acetophenon-O-(1-ethoxy-1-methylethyl)oxim als E/Z-Isomerengemisch in Form eines gelben Oels. Bei der chromatographischen Trennung an Silicagel mit n-Hexan/Ethylacetat (3:1) erhält man zuerst das E-Isomere [$^1$H-NMR ($CDCl_3$, 200 MHz): 1,428 ppm (s, -C($\underline{H_3}$)$_2$)] in Form von leicht gelblichen Oelen.

Beispiele 7-41

Analog dem in Beispiel 1, 2, 3, 4, 5 oder 6 beschriebenen Verfahren werden aus den entsprechenden Ausgangsmaterialien die in der nachstehenden Tabelle 1 aufgeführten Verbindungen der Formel I hergestellt.

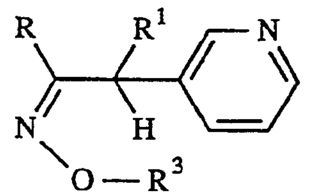

I

Tabelle 1

| Beispiel | R | $R^1$ | $R^3$ | Physikalische Daten | Methode des Beispiels |
|---|---|---|---|---|---|
| 7 | Phenyl | Wasserstoff | Wasserstoff | weisser Feststoff | 2 |
| 8 | 2,4-Dichlorphenyl | Wasserstoff | Wasserstoff | weisser Feststoff | 2 |
| 9 | α,α,α-Trifluor-o-tolyl | Wasserstoff | Wasserstoff | weisser Feststoff | 2 |
| 10 | 2,4-Dimethyl-phenyl | Wasserstoff | Wasserstoff | gelbe Flüssigkeit | 2 |
| 11 | 2-Chlor-6-fluor-phenyl | Wasserstoff | Wasserstoff | farbloser Feststoff | 2 |
| 12 | 2,6-Difluorphenyl | Wasserstoff | Wasserstoff | hellgelber Feststoff | 2 |
| 13 | 2-Fluor-4-chlor-phenyl | Wasserstoff | Wasserstoff | hellgelber Feststoff | 2 |
| 14 | 2,4-Difluorphenyl | Wasserstoff | Wasserstoff | gelber Feststoff | 2 |
| 15 | 4-Fluor-2-chlor-phenyl | Wasserstoff | Wasserstoff | weisslicher Feststoff | 2 |
| 16 | 2,4-Dichlorphenyl | Wasserstoff | Methyl | Flüssigkeit | 1,4 |
| 17 | 2,4-Dichlorphenyl | Wasserstoff | Ethyl | Flüssigkeit | 1,4 |
| 18 | 2,4-Dichlorphenyl | Wasserstoff | Isopropyl | farblose Flüssigkeit | 2 |
| 19 | 2,4-Dichlorphenyl | Wasserstoff | tert.Butyl | gelbe Flüssigkeit | 2 |
| 20 | p-Tolyl | Wasserstoff | Methyl | leicht gelbe Flüssigkeit | 2 |
| 21 | 2-Fluor-4-chlor-phenyl | Wasserstoff | Methyl | gelbliche Flüssigkeit | 2 |
| 22 | 2,4-Dichlorphenyl | Methyl | Methyl | | 3 |
| 23 | 2,4-Dichlorphenyl | Wasserstoff | 1-Methoxy-1-methylethyl | gelbe Flüssigkeit | 5 |
| 24 | o-Tolyl | Wasserstoff | 1-Methoxy-1-methylethyl | gelbe Flüssigkeit | 5 |

Tabelle 1 (Fortsetzung)

| Beispiel | R | $R^1$ | $R^3$ | Physikalische Daten | Methode des Beispiels |
|---|---|---|---|---|---|
| 25 | 2,4-Dimethylphenyl | Wasserstoff | 1-Methoxy-1-methylethyl | gelbe Flüssigkeit | 5 |
| 26 | 2-Chlor-6-fluorphenyl | Wasserstoff | 1-Methoxy-1-methylethyl | gelbe Flüssigkeit | 5 |
| 27 | 2,6-Difluorphenyl | Wasserstoff | 1-Methoxy-1-methylethyl | gelbe Flüssigkeit | 5 |
| 28 | 4-Chlor-2-fluorphenyl | Wasserstoff | 1-Methoxy-1-methylethyl | gelbe Flüssigkeit | 5 |
| 29 | 2,4-Difluorphenyl | Wasserstoff | 1-Methoxy-1-methylethyl | rötliche Flüssigkeit | 5 |
| 30 | 4-Chlor-2-fluorphenyl | Wasserstoff | 1-(n-Butoxy)-1-methylethyl | gelbe Flüssigkeit | 6 |
| 31 | 2-Chlor-4-fluorphenyl | Wasserstoff | 1-Ethyl-1-methoxybutyl | gelbe Flüssigkeit | 6 |
| 32 | 2-Chlor-4-fluorphenyl | Wasserstoff | 1-(n-Butoxy)-1-methylethyl | gelbe Flüssigkeit | 6 |
| 33 | 4-Chlor-2-fluorphenyl | Wasserstoff | 1-Ethyl-1-methoxybutyl | gelbe Flüssigkeit | 6 |
| 34 | 2,4-Dichlorphenyl | Wasserstoff | Benzyl | gelbe Flüssigkeit | 2 |
| 35 | 2,4-Dichlorphenyl | Wasserstoff | 2,6-Dichlorbenzyl | gelbe Flüssigkeit | 2 |
| 36 | 2-Fluor-6-chlorphenyl | Wasserstoff | Benzyl | gelbe Flüssigkeit | 2 |
| 37 | 2,4-Dichlorphenyl | Wasserstoff | 4-Nitrobenzyl | gelbe Flüssigkeit | 2 |
| 38 | 2,4-Dichlorphenyl | Wasserstoff | Allyl | gelbe Flüssigkeit | 2 |
| 39 | 2,4-Dichlorphenyl | Wasserstoff | Propargyl | gelbe Flüssigkeit | 2 |
| 40 | 2,4-Dichlorphenyl | Methyl | Propargyl | gelbe Flüssigkeit | 3 |
| 41 | 2,4-Dichlorphenyl | Wasserstoff | Phenyl | Flüssigkeit | 2 |

Beispiel 42

Zu einer Lösung von 2,81 g 2',4'-Dichlor-2-(3-pyridyl)-acetophenonoxim in 20 ml Dimethylformamid gibt man unter Rühren 0,55 g Natriumhydrid-Dispersion (50% in Oel) und anschliessend 2,20 g 2-(4-Fluorphenoxy)-ethylbromid zu. Das Reaktionsgemisch wird auf 100°C erwärmt und 1 Stunde bei dieser Temperatur gerührt. Dann wird auf Raumtemperatur abgekühlt und auf Eis gegossen. Man extrahiert das wässrige Gemisch mit Methylenchlorid, wäscht die organische Phase zweimal mit Wasser, trocknet sie über wasserfreiem Magnesiumsulfat und dampft sie unter vermindertem Druck ein. Nach Chromatographie an Kieselgel unter Verwendung von n-Hexan/Ethylacetat (7:3) als Laufmittel erhält man das 2',4'-Dichlor-2-(3-pyridyl)-acetophenon-O-[2-(4-fluorphenoxy)-ethyl]-oxim als E/Z-Isomerengemisch in Form eines leicht gelben Oels.

Beispiel 43

Eine Lösung von 1 g 2',4'-Dichlor-2-(3-pyridyl)-acetophenon-O-methyloxim in 20 ml Xylol wird bei Raumtemperatur mit 0,61 g 1,1-Dimethoxycyclohexan und einer Spatelspitze fein pulverisiertem Amberlyst[R]A15 versetzt, und das Ganze wird gut gerührt. Anschliessend wird 24 Stunden bei 100°C Innentemperatur nachgerührt. Nach leichtem Abkühlen werden nochmals 0,61 g 1,1-Dimethoxycyclohexan und eine Spatelspitze Amberlyst[R]A15 zugegeben. Man heizt das Gemisch auf 120°C auf und rührt es weitere 10 Stunden.

Nach Abkühlen auf Raumtemperatur wird über Celite[R] abfiltriert und das Filtrat in Diethylether aufgenommen. Die etherische Lösung wird mit Wasser gewaschen, über wasserfreiem Magnesiumsulfat getrocknet und unter vermindertem Druck eingeengt, und der Rückstand (1,62 g) an Kieselgel unter Verwendung von n-Hexan/Ethylacetat (7:3) chromatographisch gereinigt. Man erhält zuerst das (E)-2',4'-Dichlor-2-(3-pyridyl)-acetophenon-O-(1-methoxy-cyclohexyl)-oxim [$^1$H-NMR ($CDCl_3$, 200 MHz): 4,114 ppm (s, $C\underline{H}_2$-$C_5H_4N$)] und dann das (Z)-2',4'-Dichlor-2-(3-pyridyl)-acetophenon-O-(1-methoxycyclohexyl)oxim [$^1$H-NMR ($CDCl_3$, 200 MHz): 3,882 ppm (s, -$C\underline{H}_2$-$C_5H_4N$)] als gelbe Festkörper.

Beispiel 44

Eine Lösung von 10 g 2',4'-Dichlor-2-(3-pyridyl)-acetophenon-O-methyloxim in 100 ml Chloroform wird bei 0°C mit 7,2 g m-Chlorperbenzoesäure versetzt und anschliessend 24 Stunden im Kühlschrank bei ca. 4°C aufbewahrt. Dann wird mit 200 ml Chloroform verdünnt und mit 250 ml 10%-iger Kaliumcarbonatlösung extrahiert. Die organische Phase wird über wasserfreiem Natriumsulfat getrocknet und eingeengt. Als Rückstand erhält man das 2',4'-Dichlor-2-(3-pyridyl)-acetophenon-O-methyloxim-1-oxid als E,Z-Isomerengemisch in Form einer gelblichen Paste.

Tabelle 2 enthält weitere Beispiele von Verbindungen der Formel I. Bei diesen Verbindungen ist jeweils die zur Herstellung verwendete Verfahrensvariante des in der vorliegenden Anmeldung für die Verbindungen der Formel I beschriebenen Herstellungsverfahrens angegeben.

| Beisp. | Isomerenform | Struktur | Physikalische Daten | Methode |
|---|---|---|---|---|
| 45 | E/Z | 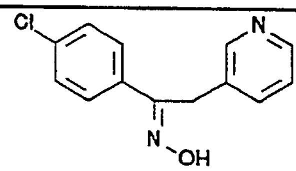 | 136-137 °C (Smp.) | a |
| 46 | E/Z | 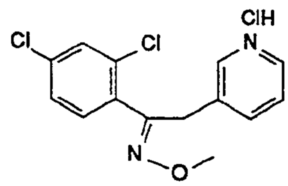 | hellgelber Festkörper | a |
| 47 | E/Z | 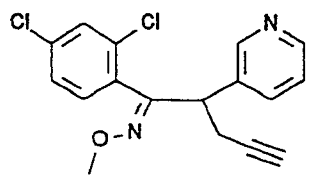 | dunkelrote Flüssigkeit | a |
| 48 | E/Z | 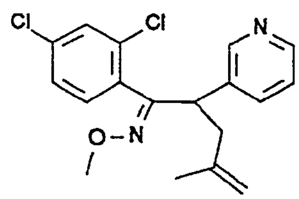 | orange Flüssigkeit | a |
| 49 | E/Z | 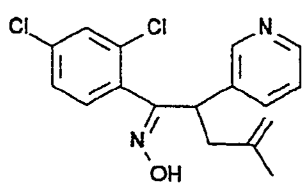 | 134-138 °C (Smp.) | a |
| 50 | E/Z | 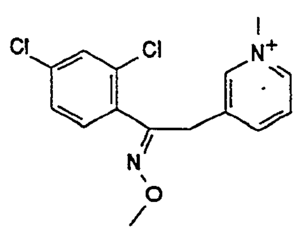 | 175-179 °C (Smp.) | a |
| 51 | E/Z | 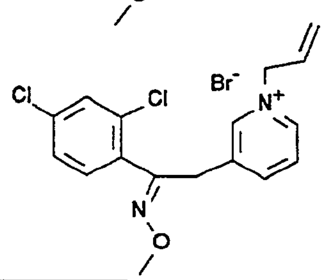 | 175-179 °C (Smp.) | a |

| Beisp. | Isomerenform | Struktur | Physikalische Daten | Methode |
|---|---|---|---|---|
| 52 | E/Z | 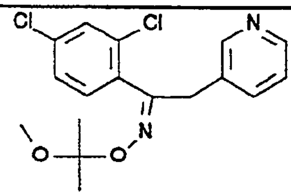 | gelbe Flüssigkeit | d |
| 53 | E | 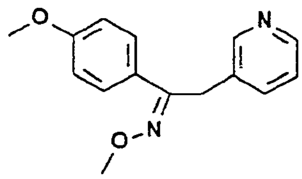 | leicht gelbliche Flüssigkeit | a |
| 54 | E/Z | 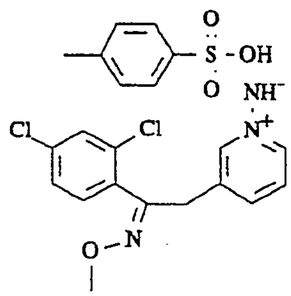 | grauer Festkörper | a |
| 55 | E/Z | 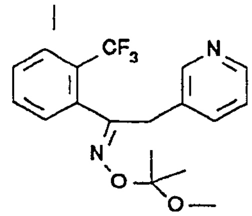 | gelbliche Flüssigkeit | d |
| 56 | E | 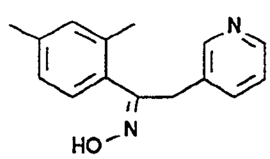 | braune Kristalle | b |
| 57 | E/Z | 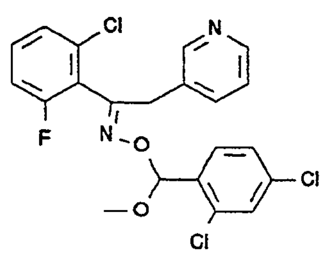 | gelbe Flüssigkeit | d |
| 58 | E/Z | 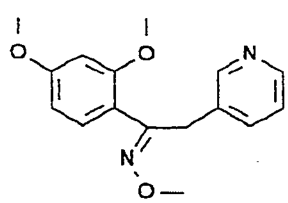 | Sdp. 170 C/0.03 Torr, gelbe Flüssigkeit | a |

| Beisp. | Isomerenform | Struktur | Physikalische Daten | Methode |
|---|---|---|---|---|
| 59 | E/Z | 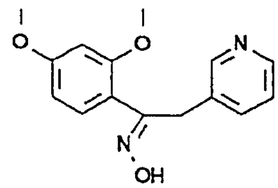 | 139-141.5 °C, beige Kristalle | b |
| 60 | E/Z | 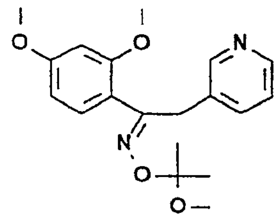 | braunes Harz | d |
| 61 | E/Z | 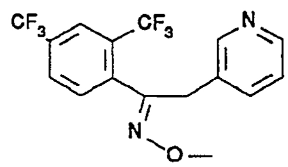 | gelber Festkörper | a |
| 62 | E/Z | 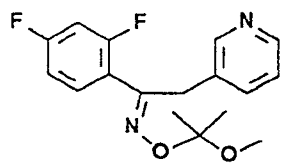 | blassgelbes Oel | d |
| 63 | E/Z | 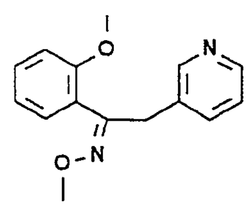 | farblose Flüssigkeit | a |
| 64 | Z | 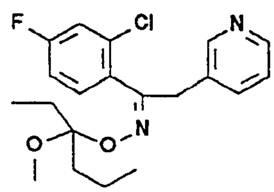 | gelbe Flüssigkeit | d |
| 65 | Z | 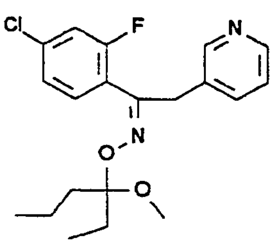 | gelbe Flüssigkeit | d |

| Beisp. | Isomerenform | Struktur | Physikalische Daten | Methode |
|---|---|---|---|---|
| 66 | E/Z | 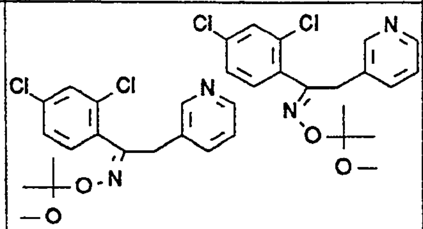 | gelbe Flüssigkeit | d |
| 67 | E | 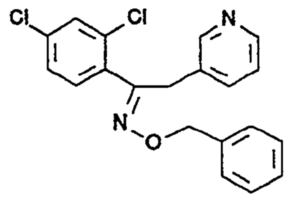 | gelbe Flüssigkeit | b |
| 68 | Z | 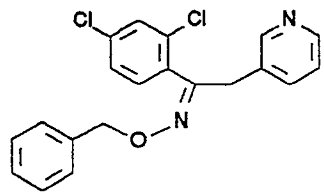 | gelbe Flüssigkeit | b |
| 69 | E/Z | 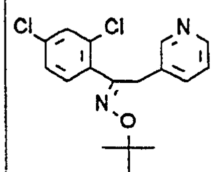 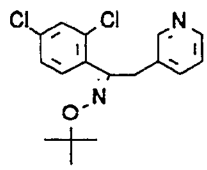 | gelbe Flüssigkeit | b |
| 70 | E | 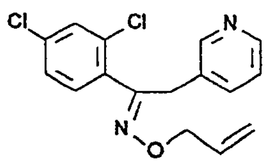 | gelbe Flüssigkeit | b |
| 71 | Z | 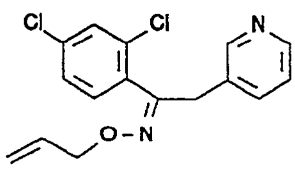 | gelbe Flüssigkeit | b |
| 72 | Z | 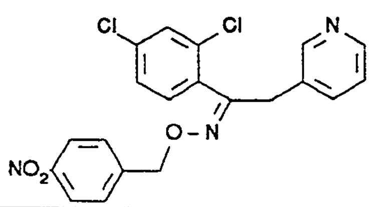 | gelbe Flüssigkeit | b |

| Beisp. | Isomerenform | Struktur | Physikalische Daten | Methode |
|---|---|---|---|---|
| 73 | E/Z | 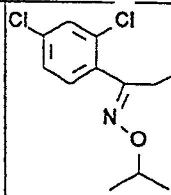 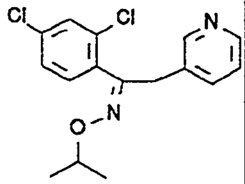 | gelbe Flüssigkeit | b |
| 74 | E/Z | 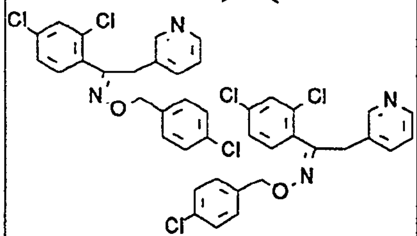 | gelbe Flüssigkeit | b |
| 75 | Z | 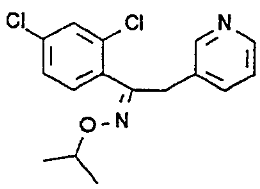 | gelbe Flüssigkeit | b |
| 76 | Z | 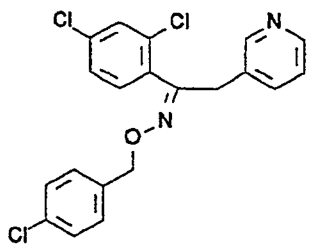 | gelbe Flüssigkeit | b |
| 77 | E | 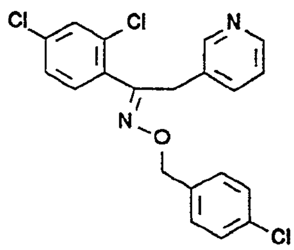 | gelbe Flüssigkeit | b |
| 78 | E | 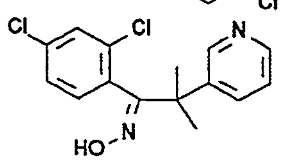 | 203-205 °C (Smp.), weisse Kristalle | b |
| 79 | E | 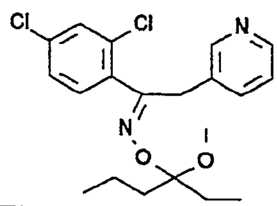 | gelbliche Flüssigkeit | d |

| Beisp. | Isomerenform | Struktur | Physikalische Daten | Methode |
|---|---|---|---|---|
| 80 | Z | 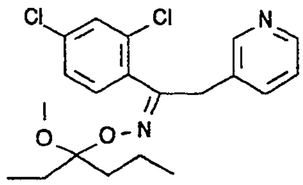 | gelbliche Flüssigkeit | d |
| 81 | E | 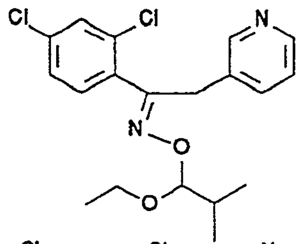 | gelbliche Flüssigkeit | d |
| 82 | Z | 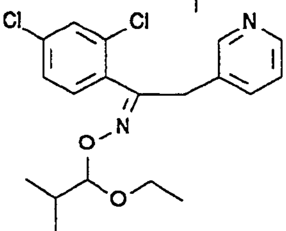 | gelbliche Flüssigkeit | d |
| 83 | E | 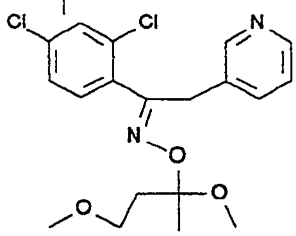 | gelbliche Flüssigkeit | d |
| 84 | E | 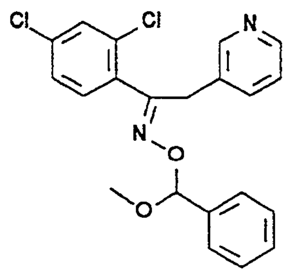 | gelbliche Flüssigkeit | d |
| 85 | E | 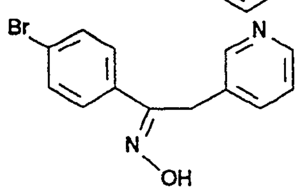 | 137-140 °C (Smp.), gelbliche Kristalle | b |
| 86 | E | 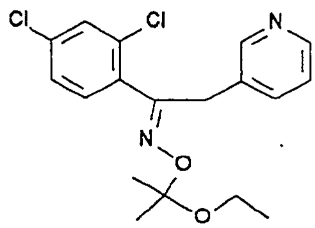 | gelbe Flüssigkeit | d |

| Beisp. | Isomerenform | Struktur | Physikalische Daten | Methode |
|---|---|---|---|---|
| 87 | Z | 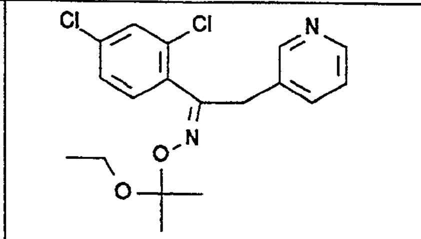 | gelber, amorpher Feststoff | d |
| 88 | E | 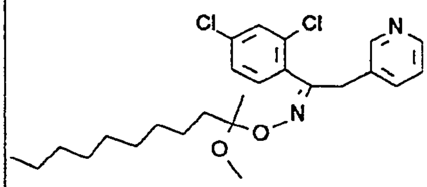 | gelbliche Flüssigkeit | d |
| 89 | Z | 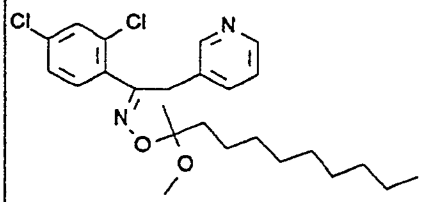 | gelbliche Flüssigkeit | d |
| 90 | E/Z | 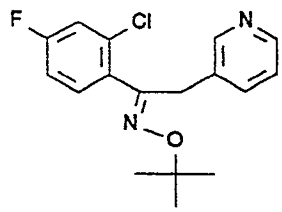 | gelbe Flüssigkeit | b |
| 91 | E | 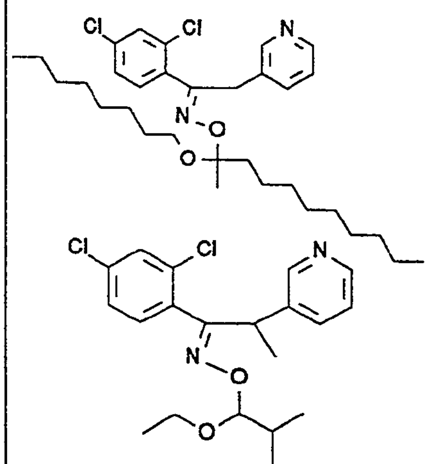 | gelbe Flüssigkeit | d |
| 92 | E/Z | | gelbe Flüssigkeit | d |
| 93 | E | 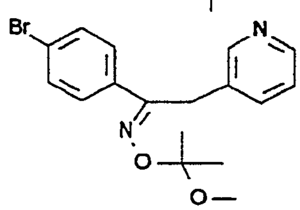 | gelbe Flüssigkeit | d |

| Beisp. | Isomerenform | Struktur | Physikalische Daten | Methode |
|---|---|---|---|---|
| 94 | E/Z | 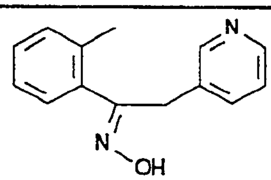 | 108-110 °C (Smp.), gelbe Kristalle | b |
| 95 | E | 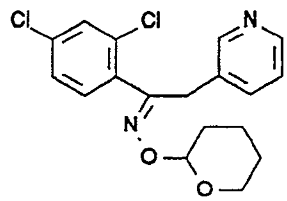 | gelbe Flüssigkeit | b |
| 96 | Z | 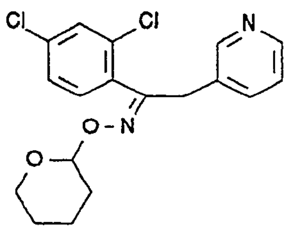 | gelbe Flüssigkeit | b |
| 97 | E | 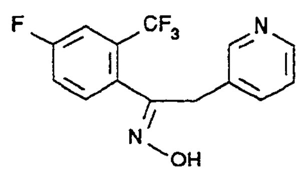 | 150-152 °C (Smp.), farblose Kristalle | b |
| 98 | E | 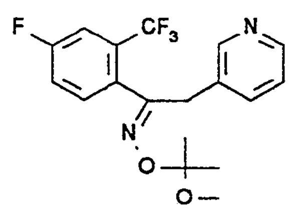 | gelbe Flüssigkeit | d |

II. Formulierungsbeispiele:

Beispiel F1:

Granulate, besonders geeignet für die Anwendung in Wasserreiskulturen, enthalten die folgenden Bestandteile:

| | | |
|---|---|---|
| Wirkstoff (Verbindung der Formel I oder N-Oxid davon oder Säureadditionssalz der Verbindung I oder des N-Oxids) | 50 | g/kg |
| Dipropylenglykol-monomethylether | 50 | g/kg |
| Montmorillonite-Trägergranulat, 24/48 mesh Grösse | 900 | g/kg |

Zur Herstellung wird der Wirkstoff mit der Trägerflüssigkeit (Dipropylenglykol-monomethylether) intensiv vermischt. Diese Lösung wird anschliessend in einem Mischer auf das vorgelegte Trägergranulat aufgesprüht, damit eine gleichmässige Imprägnierung des Trägers gewährleistet wird.

Diese Granulate werden mit einem geeigneten Granulat-Applikarionsgerät direkt ausgebracht.

Beispiel F2:

Ein Spritzpulver enthält folgende Bestandteile:

| | Gewichtsprozent |
|---|---|
| Wirkstoff (Verbindung der Formel I oder N-Oxid davon oder Säureadditionssalz der Verbindung I oder des N-Oxids) | 25 |
| Hydratisierte Kieselsäure (Hilfsträgerstoff) | 20 |
| Natrium-laurylsulfat (Netzmittel) | 2 |
| Natrium-lignosulfonat (Dispergiermittel) | 4 |
| Kaolin (Trägerstoff) | 49 |
| | --- |
| | 100 |

Es eignen sich als Wirkstoffe für diese Formulierung insbesondere diejenigen Verbindungen I, N-Oxide bzw. Säureadditionssalze, die bei Raumtemperatur flüssig sind oder einen relativ niederen Schmelzpunkt, also etwa 100°C oder weniger, besitzen.

Zur Herstellung wird vorerst der flüssige bzw. geschmolzene Wirkstoff in einem Pulvermischer auf die vorgelegte Kieselsäure aufgedüst. Anschliessend werden die weiteren Bestandteile zugemischt, und das Ganze wird unter Verwendung einer Stiftenmühle oder vergleichbaren Mahleinrichtung fein gemahlen.

Das resultierende Spritzpulver ergibt beim Einrühren in Wasser eine feine Suspension gewünschter Konzentration, die sich als gebrauchsfertige Spritzbrühe eignet.

Beispiel F3:

Ein emulgierbares Konzentrat enthält folgende Bestandteile

| | | | |
|---|---|---|---|
| Wirkstoff (Verbindung der Formel I oder N-Oxid davon oder Säureadditionssalz der Verbindung I oder des N-Oxids) | | 250 | g/l |
| Nonylphenolpolyethoxylat (nichtionogener Emulgator) | | 50 | g/l |
| Calcium-dodecylbenzolsulfonat (anionischer Emulgator) | | 25 | g/l |
| Gemisch von Alkylbenzolen (Lösungsmittel) | ad | 1000 | ml |

Der Wirkstoff und die Emulgatoren werden unter Rühren im Lösungsmittel gelöst. Das resultierende emulgierbare Konzentrat lässt sich in Wasser emulgieren und ergibt so eine gebrauchsfertige Spritzbrühe mit der gewünschten Konzentration.

Biologische Beispiele

Beispiel B1: Pre-emergente Herbizid-Wirkung

Im Gewächshaus wird unmittelbar nach der Einsaat der Versuchspflanzen (eine Anzahl Unkräuter, sowohl monokotyle wie dikotyle) in Saatschalen die Erdoberfläche mit einer wässrigen Spritzbrühe entsprechend einer Aufwandmenge von 3 kg Wirksubstanz/Hektar behandelt.

Die Test-Substanzen werden vorzugsweise als emulgierbares Konzentrat (EC) formuliert und unmittelbar vor der Applikation in Wasser auf die gewünschte Konzentration verdünnt. Unlösliche Substanzen werden mittels Kaolin als inertem Träger als benetzbares Pulver (WP) formuliert. Dieses wird unmittelbar vor der Applikation in Wasser suspendiert.

Die Dosierungen in g Aktivsubstanz/ha beziehen sich auf die Erdoberfläche in den Behältern, soweit nicht anders angegeben. Das Sprühvolumen beträgt 500 L/ha.

Die Samen der Pflanzen werden in Plastik-Pflanztöpfe verschiedener Grösse mit hitzesterilisierter (gedämpfter) Erde gesät (Landerde 2.6% Torf, 20% Ton, 30% Schluff, 47% Sand). Die Pflanzen werden im Gewächshaus bei mittlerer Temperatur (17 - 25°C im Winter, 18 - 35°C im Sommer) gehalten (Luftfeuchte 30 - 90%). Die Länge der Photoperiode beträgt 13 bis 16 Stunden/Tag, falls erforderlich, wird künstliches Licht (15000 bis 18000 Lux) zugeschaltet. Die künstliche Beleuchtung wird auch bei unzureichender Tageslicht-Intensität automatisch aktiviert.

Nach 3 Wochen wird die Herbizidwirkung mit einem zehnstufigen (10 = vollständige Schädigung, 0 = keine Wirkung) linearen Boniturschema im Vergleich zu einer unbehandelten Kontrollgruppe bewertet.

In diesem Versuch zeigen die in den Beispielen beschriebenen Verbindungen der Formel I starke Herbizidwirkung.

Beispiel B2: Post-emergente Herbizid-Wirkung (Kontaktherbizid)

Eine Anzahl Unkräuter, sowohl monokotyle wie dikotyle, wurden nach dem Auflaufen (im 4- bis 6-Blattstadium) mit einer wässrigen Wirkstoffdispersion in einer Dosierung von 3 kg Wirksubstanz pro Hektar behandelt.

Die Test-Substanzen werden vorzugsweise als emulgierbares Konzentrat (EC) formuliert und unmittelbar vor der Applikation in Wasser auf die gewünschte Konzentration verdünnt. Unlösliche Substanzen werden mittels Kaolin als inertem Träger als benetzbares Pulver (WP) formuliert. Dieses wird unmittelbar vor der Applikation in Wasser suspendiert.

Die Dosierungen in g Aktivsubstanz/ha beziehen sich auf die Erdoberfläche in den Behältern, soweit nicht anders angegeben. Das Sprühvolumen beträgt 500 L/ha.

Die Samen der Pflanzen werden in Plastik-Pflanztöpfe verschiedener Grösse mit hitzesterilisierter (gedämpfter) Erde gesät ('Optima' Erde 80% Torf, 20% Löss). Die Pflanzen werden im Gewächshaus bei mittlerer Temperatur ( 17 - 25°C im Winter, 18 - 35°C im Sommer) gehalten (Luftfeuchte 30 - 90%). Die Länge der Photoperiode beträgt 13 bis 16 Stunden/Tag, falls erforderlich, wird künstliches Licht ( 15000 bis 18000 Lux) zugeschaltet. Die künstliche Beleuchtung wird auch bei unzureichender Tageslicht-Intensität automatisch aktiviert.

Nach 3 Wochen wird die Herbizidwirkung mit einem zehnstufigen (10 = vollständige Schädigung, 0 = keine

Wirkung) linearen Boniturschema im Vergleich zu einer unbehandelten Kontrollgruppe bewertet.

Auch in diesem Versuch zeigen die in den Beispielen beschriebenen Verbindungen starke Herbizidwirkung.

Beispiel B3: Herbizidwirkung für Wasserreis (paddy)

Die Samen der Pflanzen werden in unten geschlossene Plastikbecher oder -boxen verschiedener Grösse mit gedüngter Erde gesät. Die Erde ist hitzesterilisiert (gedämpft) und enthält etwa 80% (w/v) Lehm und 20% Torf sowie 0.05% Nutricote (16/10/10) und 0.05% 'Plantamaag' als Dünger. Die Pflanzen werden in einem speziellen Gewächshaus-Abteil mit hoher Temperatur (20 - 35°C) und hoher Luftfeuchtigkeit (60 - 80%) gehalten, letztere wird mit einer Sprinkler-Anlage konstant gehalten. Die Länge der Photoperiode beträgt 13 bis 16 Stunden/Tag, falls erforderlich wird künstliches Licht (15000 bis 18000 Lux) zugeschaltet. Die künstliche Beleuchtung wird auch bei unzureichender Tageslicht-Intensität automatisch aktiviert.

Die Test-Substanzen werden vorzugsweise als emulgierbares Konzentrat (EC) formuliert und unmittelbar vor der Applikation in Wasser auf die gewünschte Konzentration verdünnt. Unlösliche Substanzen werden mittels Kaolin als inertem Träger als benetzbares Pulver (WP) formuliert. Dieses wird unmittelbar vor der Applikation in Wasser suspendiert.

Die Dosierungen in g Aktivsubstanz/ha beziehen sich auf die Erdoberfläche in den Behältern, soweit nicht anders angegeben. Das Sprühvolumen beträgt 500 L/ha.

Die Versuche werden nach 3 oder 4 Wochen (Nachauflauf-, bzw. Vorauflauf-Behandlung) mit einem zehnstufigen ( 10 = vollständige Schädigung, 0 = keine Wirkung) linearen Boniturschema im Vergleich zu einer unbehandelten Kontrollgruppe bewertet.

Beispiele für die gute selektive Herbizidwirkung der in den Beispielen der vorliegenden Anmeldung beschriebenen Verbindungen der Formel I sind in der Tabelle 3 aufgeführt:

Tabelle 3: Herbizide Wirkung im Wasserreis (Vorauflauf), Aufwandmenge 3kg/ha:

| Beispiel: | Isomerenform: | Echinochloa crus-galli | Reis Oryza sativa |
|---|---|---|---|
| 6 | E/Z | 9 | 0 |
| 17 | | 10 | 0 |
| 20 | E | 10 | 1 |
| 31 | Z | 10 | 0 |
| 50 | E | 10 | 1 |
| 69 | E/Z | 10 | 0 |
| 73 | E/Z | 10 | 0 |
| 77 | E/Z | 10 | 0 |
| 80 | E/Z | 10 | 1 |
| 97 | E/Z | 10 | 1 |

**Patentansprüche**

1. Unkrautbekämpfungsmittel, dadurch gekennzeichnet, dass es eine wirksame Menge mindestens einer Verbindung der allgemeinen Formel

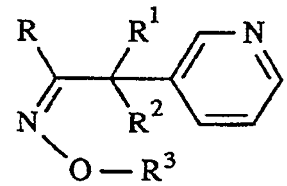

I

worin
R eine gegebenenfalls substituierte Phenylgruppe der Formel

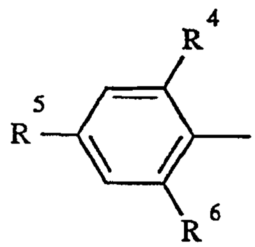

(a)

$R^1$ und $R^2$ unabhängig voneinander Wasserstoff, $C_n$-Alkyl, $C_n$-Alkenyl oder $C_n$-Alkinyl, wobei n eine Zahl von 1-4 bedeutet und $R^1$ oder $R^2$ für Wasserstoff stehen, wenn n grösser als 2 ist,
$R^3$ Wasserstoff; $C_{1-10}$-Alkyl; $C_{3-10}$-Alkoxyalkyl oder $C_{3-10}$-Alkylthioalkyl mit je einer Alkylenkette von mindestens 2 Kohlenstoffatomen; Aryl; Aryl-$C_{1-3}$-alkyl; $C_{3-6}$-Alkenyl; $C_{3-6}$-Alkinyl; oder eine Gruppe

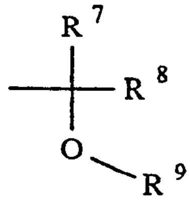

(b)

$R^4$ Wasserstoff oder Fluor,
$R^5$ und $R^6$ unabhängig voneinander Wasserstoff, Halogen, $C_{1-4}$-Alkyl oder Trifluormethyl,
$R^7$ und $R^8$ unabhängig voneinander Wasserstoff, $C_{1-6}$-Alkyl, $C_{3-6}$-Alkenyl oder $C_{3-6}$-Alkinyl
oder
$R^7$ und $R^8$ zusammen gegebenenfalls mit $C_{1-3}$-Alkyl mono- oder mehrfach substituiertes Tetra- oder Pentamethylen,
und
$R^9$ $C_{1-10}$-Alkyl; $C_{3-6}$-(Alkoxy-alkyl) oder $C_{3-6}$-(Alkylthio-alkyl) mit je einer Alkylenkette von mindestens zwei Kohlenstoffatomen; Aryl-$C_{1-3}$-alkyl; gegebenenfalls mit $C_{1-3}$-Alkyl mono- oder mehrfach substituiertes $C_{3-6}$-Cycloalkyl; $C_{3-10}$-Alkenyl; $C_{3-10}$-Alkinyl; Aryl,
oder
$R^7$ Wasserstoff
und
$R^8$ und $R^9$ zusammen gegebenenfalls mit $C_{1-3}$-Alkyl mono- oder mehrfach substituiertes Tetramethylen bedeuten,
wobei die Gruppe (b) die schon oben in $R^3$ definierte $C_{3-10}$-Alkoxyalkylgruppe nicht umfassen soll,
deren N-Oxide sowie die Säureadditionssalze der Verbindungen I und der entsprechenden N-Oxide enthält.

2. Unkrautbekämpfungsmittel nach Anspruch 1, worin R 2-Chlor-4-fluorphenyl, 4-Chlor-2-fluorphenyl, 2,4-Dichlorphenyl, 2-Chlor-6-fluorphenyl oder 2,6-Difluorphenyl bedeutet.

3. Unkrautbekämpfungsmittel nach Anspruch 1 oder 2, worin $R^1$ oder $R^2$ Wasserstoff und $R^2$ bzw. $R^1$ Wasserstoff oder $C_{1-4}$-Alkyl bedeuten.

4. Unkrautbekämpfungsmittel nach Anspruch 1, worin $R^3$ tert.Butyl oder eine Gruppe (b) bedeutet.

5. Unkrautbekämpfungsmittel nach Anspruch 1, worin $R^3$ tert.Butyl oder eine Gruppe (b) bedeutet, in der $R^7$, $R^8$ und $R^9$ jeweils Methyl oder $R^7$ Aethyl, $R^8$ n-Propyl und $R^9$ Methyl bedeuten.

6. Unkrautbekämpfungsmittel nach Anspruch 1, dadurch gekennzeichnet, dass es als Verbindung(en) der Formel I mindestens eine aus der Gruppe
2',4'-Dichlor-2-(3-pyridyl)-acetophenon-O-(tert.butyl)-oxim,
2',4'-Dichlor-2-(3-pyridyl)-acetophenon-O-(1-methoxy-1-methylethyl)oxim,
2'-Chlor,4'-fluor-2-(3-pyridyl)-acetophenon-O-(1-methoxy-1-methylethyl)oxim
2',6'-Difluor-2-(3-pyridyl)-acetophenon-O-(1-methoxy-1-methylethyl)oxim
4'-Chlor,2'-fluor-2-(3-pyridyl)-acetophenon-O-(1-methoxy-1-methylethyl)oxim und
2'-Chlor,4'-fluor-2-(3-pyridyl)-acetophenon-O-(1-ethyl-1-methoxy-n-butyl)oxim
ausgewählte Verbindung enthält.

7. Unkrautbekämpfungsmittel nach Anspruch 1, dadurch gekennzeichnet, dass es als Verbindung(en) der Formel I mindestens eine aus der Gruppe
2',4'-Dichlor-2-methyl-2-(3-pyridyl)-propiophenon-O-(1-methox y-1-methylethyl)oxim,
2'-Chlor,4'-fluor-2-methyl-2-(3-pyridyl)-propiophenon-O-(1-methoxy-1-methylethyl)oxim,
2',4'-Dichlor-2-(3-pyridyl)-acetophenon-O-(1-ethoxyethyl)oxim,
2'-Chlor,4'-fluor-2-(3-pyridyl)-acetophenon-O-(1-ethoxyethyl)oxim,
2',4'-Dichlor-2-(3-pyridyl)-acetophenon-O-(1-methoxyethyl)oxim,
2'-Chlor,4'-fluor-2-(3-pyridyl)-acetophenon-O-(1-methoxyethyl)oxim,
2',4'-Dichlor-2-(3-pyridyl)-acetophenon-O-(1-methoxypropyl)oxim,
2'-Chlor,4'-fluor-2-(3-pyridyl)-acetophenon-O-(1-methoxypropyl)oxim,
2',4'-Dichlor-2-(3-pyridyl)-propiophenon-O-methoxymethyloxim,
2'-Chlor,4'-fluor-2-(3-pyridyl)-propiophenon-O-methoxymethyloxim,
2',4'-Dichlor-2-(3-pyridyl)-acetophenon-O-(1-methoxycyclohexyl)oxim,
2',4'-Dichlor-2-(3-pyridyl)-acetophenon-O-(2-tetrahydropyranyl)oxim und
2'-Chlor,4'-fluor-2-(3-pyridyl)-acetophenon-O-(2-tetrahydropyranyl)oxim
ausgewählte Verbindung enthält.

8. Unkrautbekämpfungsmittel nach Anspruch 1, dadurch gekennzeichnet, dass es 0,001 bis 99 Gewichtsprozent eines oder mehrerer Wirkstoffe der Formel I, 1-99 % eines festen oder flüssigen Zusatzstoffes und 0 bis 25 % eines Tensids enthält.

9. Verfahren zur Bekämpfung von Unkräutern, dadurch gekennzeichnet, dass man das gegen Unkraut zu schützende Gut und/oder die Unkräuter mit einer wirksamen Menge mindestens einer in Anspruch 1 beschriebenen Verbindung bzw. eines Mittels gemäss Anspruch 1 behandelt.

10. Verfahren gemäss Anspruch 9, dadurch gekennzeichnet, dass man eine Wirkstoffmenge zwischen 0,01 und 3 kg pro Hektar appliziert.

11. Verfahren gemäss Anspruch 9, zur selektiven pre- oder post-emergenten Bekämpfung von Unkräutern in Nutzpflanzenkulturen.

12. Verfahren gemäss Anspruch 9, zur selektiven pre- oder post-emergenten Bekämpfung von Unkräutern in Kulturen von Reis, Weizen, Mais, Zuckerrübe, Raps, Soja und Baumwolle.

13. Verfahren gemäss Anspruch 9, zur selektiven pre- oder post-emergenten Bekämpfung von Unkräutern in Wasserreiskulturen

14. Verwendung von in Anspruch 1 beschriebenen Verbindungen der Formel I oder eines Mittels gemäss Anspruch 1 zur Bekämpfung von Unkräutern.

15. Verbindungen der allgemeinen Formel

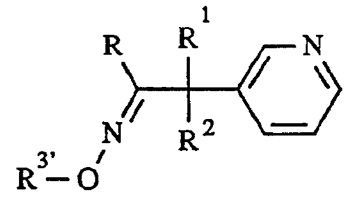

I'

worin

R eine gegebenenfalls substituierte Phenylgruppe der Formel

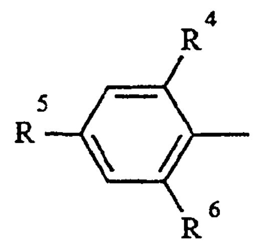

(a)

$R^1$ und $R^2$ unabhängig voneinander Wasserstoff, $C_n$-Alkyl, $C_n$-Alkenyl oder $C_n$-Akinyl, wobei n eine Zahl von 1-4 bedeutet und $R^1$ oder $R^2$ für Wasserstoff stehen, wenn n grösser als 2 ist,

$R^{3'}$ $C_{3-10}$-(Alkylthio-alkyl) mit einer Alkylenkette von mindestens zwei Kohlenstoffatomen, oder eine Gruppe

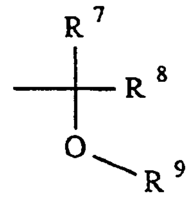

(b)

$R^4$ Wasserstoff oder Fluor,

$R^5$ und $R^6$ unabhängig voneinander Wasserstoff, Halogen, $C_{1-4}$-Alkyl oder Trifluormethyl,

$R^7$ und $R^8$ unabhängig voneinander Wasserstoff, $C_{1-6}$-Alkyl, $C_{3-6}$-Alkenyl oder $C_{3-6}$-Alkinyl

oder

$R^7$ und $R^8$ zusammen gegebenenfalls mit $C_{1-3}$-Alkyl mono- oder mehrfach substituiertes Tetra- oder Pentamethylen,

und

$R^9$ $C_{1-10}$-Alkyl; $C_{3-6}$-(Alkoxy-alkyl) oder $C_{3-6}$-(Alkylthio-alkyl) mit je einer Alkylenkette von mindestens zwei Kohlenstoffatomen; Aryl-$C_{1-3}$-alkyl; gegebenenfalls mit $C_{1-3}$-Alkyl mono- oder mehrfach substituiertes $C_{3-6}$-Cycloalkyl; $C_{3-10}$-Alkenyl; $C_{3-10}$-Alkinyl; Aryl,

oder

$R^7$ Wasserstoff

und

$R^8$ und $R^9$ zusammen gegebenenfalls mit $C_{1-3}$-Alkyl mono- oder mehrfach substituiertes Tetramethylen bedeuten,

wobei die Gruppe (b) $C_{3-10}$-Alkoxyalkylgruppe nicht umfasst, deren N-Oxide sowie die Säureadditionssalze der Verbindungen I und der N-Oxide.

**16.** Verfahren zur Herstellung von Verbindungen der allgemeinen Formel

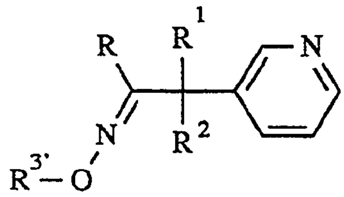

I'

worin

R eine gegebenenfalls substituierte Phenylgruppe der Formel

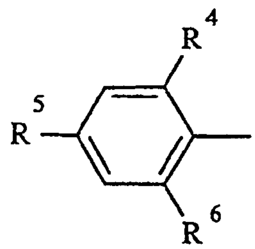

(a)

$R^1$ und $R^2$ unabhängig voneinander Wasserstoff, $C_n$-Alkyl, $C_n$-Alkenyl oder $C_n$-Alkinyl, wobei n eine Zahl von 1-4 bedeutet und $R^1$ oder $R^2$ für Wasserstoff stehen, wenn n grösser als 2 ist,

$R^{3'}$ $C_{3-10}$-(Alkylthio-alkyl) mit einer Alkylenkette von mindestens zwei Kohlenstoffatomen, oder eine Gruppe

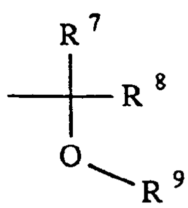

(b)

$R^4$ Wasserstoff oder Fluor,

$R^5$ und $R^6$ unabhängig voneinander Wasserstoff, Halogen, $C_{1-4}$-Alkyl oder Trifluormethyl,

$R^7$ und $R^8$ unabhängig voneinander Wasserstoff, $C_{1-6}$-Alkyl, $C_{3-6}$-Alkenyl oder $C_{3-6}$-Alkinyl

oder

$R^7$ und $R^8$ zusammen gegebenenfalls mit $C_{1-3}$-Alkyl mono- oder mehrfach substituiertes Tetra- oder Pentamethylen,

und

$R^9$ $C_{1-10}$-Alkyl; $C_{3-6}$-(Alkoxy-alkyl) oder $C_{3-6}$-(Alkylthio-alkyl) mit je einer Alkylenkette von mindestens zwei Kohlenstoffatomen; Aryl-$C_{1-3}$-alkyl; gegebenenfalls mit $C_{1-3}$-Alkyl mono- oder mehrfach substituiertes $C_{3-6}$-Cycloalkyl; $C_{3-10}$-Alkenyl; $C_{3-10}$-Alkinyl; Aryl,

oder

$R^7$ Wasserstoff

und

$R^8$ und $R^9$ zusammen gegebenenfalls mit $C_{1-3}$-Alkyl mono- oder mehrfach substituiertes Tetramethylen bedeuten,

wobei die Gruppe (b) $C_{3-10}$-Alkoxyalkylgruppe nicht umfasst, deren N-Oxide sowie die Säureadditionssalze der Verbindungen I und der N-Oxide, dadurch gekennzeichnet, dass man

a) zwecks Herstellung derjenigen Verbindungen der Formel I', in denen $R^1$ und $R^2$ Wasserstoff bedeuten, und $R^{3'}$ verschieden von einer Gruppe (b) ist, ein Enamin der allgemeinen Formel

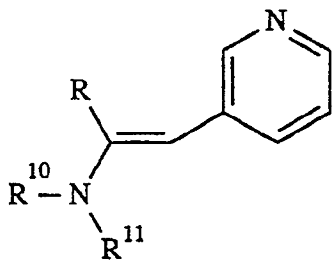

II

worin
R die oben angegebene Bedeutung besitzt
und
$R^{10}$ und $R^{11}$ unabhängig voneinander $C_{1-4}$-Alkyl, insbesondere Methyl oder Ethyl, bedeuten, mit einem O-substituierten Hydroxylamin der allgemeinen Formel

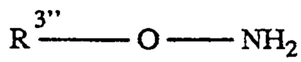

III

worin
$R^{3''}$ $C_{1-10}$-Alkyl; $C_{3-10}$-Alkoxyalkyl oder $C_{3-10}$-Alkylthioalkyl mit je einer Alkylenkette von mindestens 2 Kohlenstoffatomen; Aryl; Aryl-$C_{1-3}$-alkyl; $C_{3-6}$-Alkenyl; $C_{3-6}$-Alkinyl bedeutet,
oder mit einem anorganischen Säureadditionssalz davon umsetzt,

b) zwecks Herstellung derjenigen Verbindungen der Formel I' und von deren N-Oxiden, in denen $R^{3'}$ verschieden von einer Gruppe (b) ist, ein Acetophenonderivat oder dessen N-Oxid der allgemeinen Formel

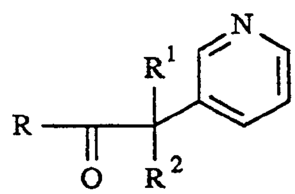

IV

worin R, $R^1$ und $R^2$ die oben angegebenen Bedeutungen besitzen, mit einem O-substituierten Hydroxylamin der oben angegebenen allgemeinen Formel III oder mit einem anorganischen Säureadditionssalz davon oder mit Hydroxylamin-hydrochlorid umsetzt,

c) zwecks Herstellung derjenigen Verbindungen der Formel I', in denen $R^{3'}$ verschieden von einer Gruppe (b) ist, und von deren N-Oxiden, ein Oxim oder dessen N-Oxid der allgemeinen Formel

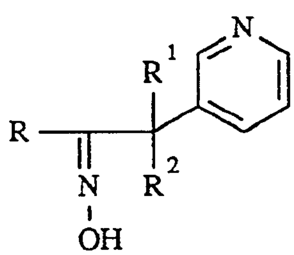

V

worin R, $R^1$ und $R^2$ die oben angegebenen Bedeutungen haben, mit einer Verbindung der allgemeinen Formel

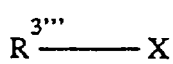

VI

worin
$R^{3'''}$ $C_{3-6}$-(Alkylthio-alkyl) oder $C_{3-8}$-(Alkoxy-alkyl) mit einer Alkylenkette von mindestens zwei Kohlenstoffatomen; Aryl-$C_{1-3}$-alkyl; gegebenenfalls mit $C_{1-3}$-Alkyl mono- oder mehrfach substituiertes $C_{3-6}$-Cycloalkyl; $C_{3-10}$-Alkenyl; $C_{3-10}$-Alkinyl;
und
X eine Abgangsgruppe, z.B. Chlor, Brom, Jod, Mesyloxy, Tosyloxy oder einen Alkylsulfatrest, bedeuten, umsetzt,

d) zwecks Herstellung derjenigen Verbindungen der Formel I' und deren N-Oxiden, in denen $R^{3'}$ eine Gruppe (b) bedeutet, in der aber $R^8$ und $R^9$ miteinander nicht verknüpft sind, ein Oxim der oben angegebenen allgemeinen Formel V oder dessen N-Oxid mit einem Diether der allgemeinen Formel

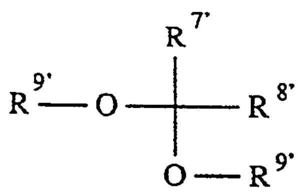

VII

worin $R^{7'}$, $R^{8'}$ und $R^{9'}$ die oben angegebenen einzelnen Bedeutungen von $R^7$, $R^8$ bzw. $R^9$ besitzen und zudem $R^{7'}$und $R^{8'}$ zusammen gegebenenfalls mit $C_{1-3}$-Alkyl mono- oder mehrfach substituiertes Tetra- oder Pentamethylen bedeuten können, umsetzt, oder

e) zwecks Herstellung derjenigen Verbindungen der Formel I' und deren N-Oxiden, in denen $R^{3'}$ eine Gruppe (b) bedeutet, in der $R^7$ Wasserstoff und $R^8$ und $R^9$ zusammen gegebenenfalls mit $C_{1-3}$-Alkyl mono- oder mehrfach substituiertes Tetramethylen bedeuten, ein Oxim der oben angegebenen allgemeinen Formel V oder dessen N-Oxid mit einer Verbindung der allgemeinen Formel

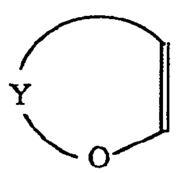

VIII

worin Y gegebenenfalls mit $C_{1-3}$-Alkyl mono- oder mehrfach substituiertes Trimethylen bedeutet, umsetzt.

**Patentansprüche für folgenden Vertragsstaat: ES**

1. Unkrautbekämpfungsmittel, dadurch gekennzeichnet, dass es eine wirksame Menge mindestens einer Verbindung der allgemeinen Formel

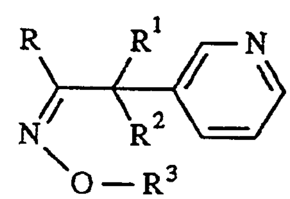

I

worin
R eine gegebenenfalls substituierte Phenylgruppe der Formel

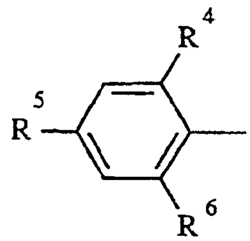 (a)

$R^1$ und $R^2$ unabhängig voneinander Wasserstoff, $C_n$-Alkyl, $C_n$-Alkenyl oder $C_n$-Alkinyl, wobei n eine Zahl von 1-4 bedeutet und $R^1$ oder $R^2$ für Wasserstoff stehen, wenn n grösser als 2 ist,
$R^3$ Wasserstoff; $C_{1-10}$-Alkyl; $C_{3-10}$-Alkoxyalkyl oder $C_{3-10}$-Alkylthioalkyl mit je einer Alkylenkette von mindestens 2 Kohlenstoffatomen; Aryl; Aryl-$C_{1-3}$-alkyl; $C_{3-6}$-Alkenyl; $C_{3-6}$-Alkinyl; oder eine Gruppe

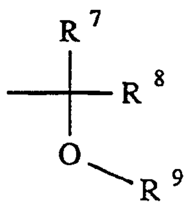 (b)

$R^4$ Wasserstoff oder Fluor,
$R^5$ und $R^6$ unabhängig voneinander Wasserstoff, Halogen, $C_{1-4}$-Alkyl oder Trifluormethyl,
$R^7$ und $R^8$ unabhängig voneinander Wasserstoff, $C_{1-6}$-Alkyl, $C_{3-6}$-Alkenyl oder $C_{3-6}$-Alkinyl
oder
$R^7$ und $R^8$ zusammen gegebenenfalls mit $C_{1-3}$-Alkyl mono- oder mehrfach substituiertes Tetra- oder Pentamethylen,
und
$R^9$ $C_{1-10}$-Alkyl; $C_{3-6}$-(Alkoxy-alkyl) oder $C_{3-6}$-(Alkylthio-alkyl) mit je einer Alkylenkette von mindestens zwei Kohlenstoffatomen; Aryl-$C_{1-3}$-alkyl; gegebenenfalls mit $C_{1-3}$-Alkyl mono- oder mehrfach substituiertes $C_{3-6}$-Cycloalkyl; $C_{3-10}$-Alkenyl; $C_{3-10}$-Alkinyl; Aryl,
oder
$R^7$ Wasserstoff
und
$R^8$ und $R^9$ zusammen gegebenenfalls mit $C_{1-3}$-Alkyl mono- oder mehrfach substituiertes Tetramethylen bedeuten,
wobei die Gruppe (b) die schon oben in $R^3$ definierte $C_{3-10}$-Alkoxyalkylgruppe nicht umfassen soll,
deren N-Oxide sowie die Säureadditionssalze der Verbindungen I und der entsprechenden N-Oxide enthält.

2. Unkrautbekämpfungsmittel nach Anspruch 1, worin R 2-Chlor-4-fluorphenyl, 4-Chlor-2-fluorphenyl, 2,4-Dichlorphenyl, 2-Chlor-6-fluorphenyl oder 2,6-Difluorphenyl bedeutet.

3. Unkrautbekämpfungsmittel nach Anspruch 1 oder 2, worin $R^1$ oder $R^2$ Wasserstoff und $R^2$ bzw. $R^1$ Wasserstoff oder $C_{1-4}$-Alkyl bedeuten.

4. Unkrautbekämpfungsmittel nach Anspruch 1, worin $R^3$ tert.Butyl oder eine Gruppe (b) bedeutet.

5. Unkrautbekämpfungsmittel nach Anspruch 1, worin $R^3$ tert.Butyl oder eine Gruppe (b) bedeutet, in der $R^7$, $R^8$ und $R^9$ jeweils Methyl oder $R^7$ Aethyl, $R^8$ n-Propyl und $R^9$ Methyl bedeuten.

6. Unkrautbekämpfungsmittel nach Anspruch 1, dadurch gekennzeichnet, dass es als Verbindung(en) der Formel I mindestens eine aus der Gruppe
2',4'-Dichlor-2-(3-pyridyl)-acetophenon-O-(tert.butyl)-oxim,
2',4'-Dichlor-2-(3-pyridyl)-acetophenon-O-(1-methoxy-1-methylethyl)oxim,
2'-Chlor,4'-fluor-2-(3-pyridyl)-acetophenon-O-(1-methoxy-1-methylethyl)oxim
2',6'-Difluor-2-(3-pyridyl)-acetophenon-O-(1-methoxy-1-methylethyl)oxim

4′-Chlor,2′-fluor-2-(3-pyridyl)-acetophenon-O-(1-methoxy-1-methylethyl)oxim und
2′-Chlor,4′-fluor-2-(3-pyridyl)-acetophenon-O-(1-ethyl-1-methoxy-n-butyl)oxim
ausgewählte Verbindung enthält.

7. Unkrautbekämpfungsmittel nach Anspruch 1, dadurch gekennzeichnet, dass es als Verbindung(en) der Formel I mindestens eine aus der Gruppe
2′,4′-Dichlor-2-methyl-2-(3-pyridyl)-propiophenon-O-(1-methox y-1-methylethyl)oxim,
2′-Chlor,4′-fluor-2-methyl-2-(3-pyridyl)-propiophenon-O-(1-methoxy-1-methylethyl)oxim,
2′,4′-Dichlor-2-(3-pyridyl)-acetophenon-O-(1-ethoxyethyl)oxim,
2′-Chlor,4′-fluor-2-(3-pyridyl)-acetophenon-O-(1-ethoxyethyl)oxim,
2′,4′-Dichlor-2-(3-pyridyl)-acetophenon-O-(1-methoxyethyl)oxim,
2′-Chlor,4′-fluor-2-(3-pyridyl)-acetophenon-O-(1-methoxyethyl)oxim,
2′,4′-Dichlor-2-(3-pyridyl)-acetophenon-O-(1-methoxypropyl)oxim,
2′-Chlor,4′-fluor-2-(3-pyridyl)-acetophenon-O-(1-methoxypropyl)oxim,
2′,4′-Dichlor-2-(3-pyridyl)-propiophenon-O-methoxymethyloxim,
2′-Chlor,4′-fluor-2-(3-pyridyl)-propiophenon-O-methoxymethyloxim,
2′,4′-Dichlor-2-(3-pyridyl)-acetophenon-O-(1-methoxycyclohexyl)oxim,
2′,4′-Dichlor-2-(3-pyridyl)-acetophenon-O-(2-tetrahydropyranyl)oxim und
2′-Chlor,4′-fluor-2-(3-pyridyl)-acetophenon-O-(2-tetrahydropyranyl)oxim
ausgewählte Verbindung enthält.

8. Unkrautbekämpfungsmittel nach Anspruch 1, dadurch gekennzeichnet, dass es 0,001 bis 99 Gewichtsprozent eines oder mehrerer Wirkstoffe der Formel I, 1-99 % eines festen oder flüssigen Zusatzstoffes und 0 bis 25 % eines Tensids enthält.

9. Verfahren zur Bekämpfung von Unkräutern, dadurch gekennzeichnet, dass man das gegen Unkraut zu schützende Gut und/oder die Unkräuter mit einer wirksamen Menge mindestens einer in Anspruch 1 beschriebenen Verbindung bzw. eines Mittels gemäss Anspruch 1 behandelt.

10. Verfahren gemäss Anspruch 9, dadurch gekennzeichnet, dass man eine Wirkstoffmenge zwischen 0,01 und 3 kg pro Hektar appliziert.

11. Verfahren gemäss Anspruch 9, zur selektiven pre- oder post-emergenten Bekämpfung von Unkräutern in Nutzpflanzenkulturen.

12. Verfahren gemäss Anspruch 9, zur selektiven pre- oder post-emergenten Bekämpfung von Unkräutern in Kulturen von Reis, Weizen, Mais, Zuckerrübe, Raps, Soja und Baumwolle.

13. Verfahren gemäss Anspruch 9, zur selektiven pre- oder post-emergenten Bekämpfung von Unkräutern in Wasserreiskulturen

14. Verwendung von in Anspruch 1 beschriebenen Verbindungen der Formel I oder eines Mittels gemäss Anspruch 1 zur Bekämpfung von Unkräutern.

15. Verfahren zur Herstellung von Verbindungen der allgemeinen Formel

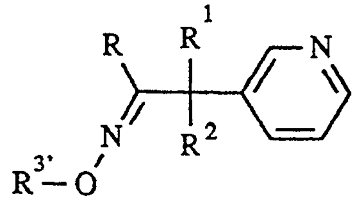

I'

worin
R eine gegebenenfalls substituierte Phenylgruppe der Formel

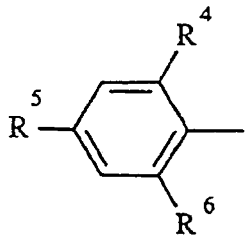

(a)

$R^1$ und $R^2$ unabhängig voneinander Wasserstoff, $C_n$-Alkyl, $C_n$-Alkenyl oder $C_n$-Alkinyl, wobei n eine Zahl von 1-4 bedeutet und $R^1$ oder $R^2$ für Wasserstoff stehen, wenn n grösser als 2 ist,
$R^{3'}$ $C_{3-10}$-(Alkylthio-alkyl) mit einer Alkylenkette von mindestens zwei Kohlenstoffatomen, oder eine Gruppe

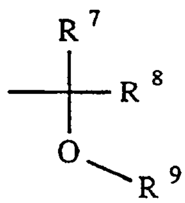

(b)

$R^4$ Wasserstoff oder Fluor,
$R^5$ und $R^6$ unabhängig voneinander Wasserstoff, Halogen, $C_{1-4}$-Alkyl oder Trifluormethyl,
$R^7$ und $R^8$ unabhängig voneinander Wasserstoff, $C_{1-6}$-Alkyl, $C_{3-6}$-Alkenyl oder $C_{3-6}$-Alkinyl
oder
$R^7$ und $R^8$ zusammen gegebenenfalls mit $C_{1-3}$-Alkyl mono- oder mehrfach substituiertes Tetra- oder Pentamethylen,
und
$R^9$ $C_{1-10}$-Alkyl; $C_{3-6}$-(Alkoxy-alkyl) oder $C_{3-6}$-(Alkylthio-alkyl) mit je einer Alkylenkette von mindestens zwei Kohlenstoffatomen; Aryl-$C_{1-3}$-alkyl; gegebenenfalls mit $C_{1-3}$-Alkyl mono- oder mehrfach substituiertes $C_{3-6}$-Cycloalkyl; $C_{3-10}$-Alkenyl; $C_{3-10}$-Akinyl; Aryl,
oder
$R^7$ Wasserstoff
und
$R^8$ und $R^9$ zusammen gegebenenfalls mit $C_{1-3}$-Alkyl mono- oder mehrfach substituiertes Tetramethylen bedeuten,
wobei die Gruppe (b) $C_{3-10}$-Akoxyalkylgruppe nicht umfasst, deren N-Oxide sowie die Säureadditionssalze der Verbindungen I und der N-Oxide, dadurch gekennzeichnet, dass man

a) zwecks Herstellung derjenigen Verbindungen der Formel I', in denen $R^1$ und $R^2$ Wasserstoff bedeuten, und $R^{3'}$ verschieden von einer Gruppe (b) ist, ein Enamin der allgemeinen Formel

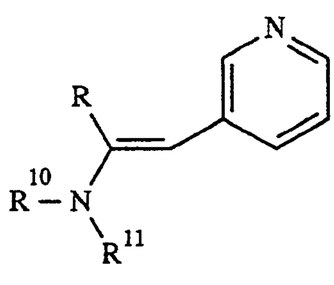

II

worin
R die oben angegebene Bedeutung besitzt
und
$R^{10}$ und $R^{11}$ unabhängig voneinander $C_{1-4}$-Akyl, insbesondere Methyl oder Ethyl, bedeuten,
mit einem O-substituierten Hydroxylamin der allgemeinen Formel

$$R^{3''}-O-NH_2 \qquad III$$

worin
$R^{3''}$ $C_{1-10}$-Alkyl; $C_{3-10}$-Alkoxyalkyl oder $C_{3-10}$-Alkylthioalkyl mit je einer Alkylenkette von mindestens 2 Kohlenstoffatomen; Aryl; Aryl-$C_{1-3}$-alkyl; $C_{3-6}$-Alkenyl; $C_{3-6}$-Alkinyl bedeutet,
oder mit einem anorganischen Säureadditionssalz davon umsetzt,

b) zwecks Herstellung derjenigen Verbindungen der Formel I' und von deren N-Oxiden, in denen $R^{3'}$ verschieden von einer Gruppe (b) ist, ein Acetophenonderivat oder dessen N-Oxid der allgemeinen Formel

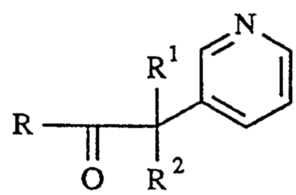

IV

worin R, $R^1$ und $R^2$ die oben angegebenen Bedeutungen besitzen, mit einem O-substituierten Hydroxylamin der oben angegebenen allgemeinen Formel III oder mit einem anorganischen Säureadditionssalz davon oder mit Hydroxylamin-hydrochlorid umsetzt,

c) zwecks Herstellung derjenigen Verbindungen der Formel I', in denen $R^{3'}$ verschieden von einer Gruppe (b) ist, und von deren N-Oxiden, ein Oxim oder dessen N-Oxid der allgemeinen Formel

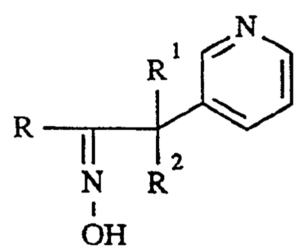

V

worin R, $R^1$ und $R^2$ die oben angegebenen Bedeutungen haben, mit einer Verbindung der allgemeinen Formel

$$R^{3'''}-X \qquad VI$$

worin
$R^{3'''}$ $C_{3-6}$-(Alkylthio-alkyl) oder $C_{3-6}$-(Alkoxy-alkyl) mit einer Alkylenkette von mindestens zwei Kohlenstoffatomen; Aryl-$C_{1-3}$-alkyl; gegebenenfalls mit $C_{1-3}$-Alkyl mono- oder mehrfach substituiertes $C_{3-6}$-Cycloalkyl; $C_{3-10}$-Alkenyl; $C_{3-10}$-Alkinyl;
und
X eine Abgangsgruppe, z.B. Chlor, Brom, Jod, Mesyloxy, Tosyloxy oder einen Alkylsulfatrest, bedeuten, umsetzt,

d) zwecks Herstellung derjenigen Verbindungen der Formel I' und deren N-Oxiden, in denen $R^{3'}$ eine Gruppe (b) bedeutet, in der aber $R^8$ und $R^9$ miteinander nicht verknüpft sind, ein Oxim der oben angegebenen allgemeinen Formel V oder dessen N-Oxid mit einem Diether der allgemeinen Formel

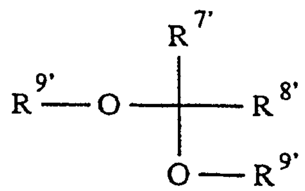

VII

worin $R^{7'}$, $R^{8'}$ und $R^{9'}$ die oben angegebenen einzelnen bedeutungen von $R^7$, $R^8$ bzw. $R^9$ besitzen und zudem $R^{7'}$ und $R^{8'}$ zusammen gegebenenfalls mit $C_{1-3}$-Alkyl mono- oder mehrfach substituiertes Tetra- oder Pentamethylen bedeuten können, umsetzt, oder

e) zwecks Herstellung derjenigen Verbindungen der Formel I' und deren N-Oxiden, in denen $R^{3'}$ eine Gruppe (b) bedeutet, in der $R^7$ Wasserstoff und $R^8$ und $R^9$ zusammen gegebenenfalls mit $C_{1-3}$-Alkyl mono- oder mehrfach substituiertes Tetramethylen bedeuten, ein Oxim der oben angegebenen allgemeinen Formel V oder dessen N-Oxid mit einer Verbindung der allgemeinen Formel

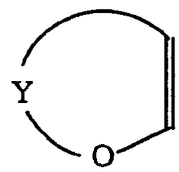

VIII

worin Y gegebenenfalls mit $C_{1-3}$-Alkyl mono- oder mehrfach substituiertes Trimethylen bedeutet, umsetzt.

Europäisches Patentamt

# EUROPÄISCHER RECHERCHENBERICHT

Nummer der Anmeldung

EP 91 81 0089

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| X | CHEMICAL ABSTRACTS, Band 100, Nr. 3, 16. Januar 1984, Seite 496, Zusammenfassung Nr. 22670d, Columbus, Ohio, US; & DE-A-3 310 148 (HOFFMAN-LA ROCHE, F. UND CO., A.-G.) 06-10-1983<br>* Zusammenfassung *<br>--- | 1-14 | A 01 N 43/40<br>C 07 D 213/53<br>C 07 D 405/12 //<br>(C 07 D 405/12<br>C 07 D 309:00<br>C 07 D 213:00 ) |
| D,X | DE-A-3 310 148 (F. HOFFMANN-LA ROCHE & CO.)<br>* Patentansprüche; Seite 12, Zeile 1 - Seite 18, Zeile 3 *<br>--- | 1-5,8 | |
| D,X | EP-A-0 049 854 (F. HOFFMANN-LA ROCHE & CO.)<br>* Seite 1, Zeile 1 - Seite 3, Zeile 22; Beispiele; Patentansprüche *<br>--- | 1-5,8 | |
| D,X | DE-A-3 309 466 (F. HOFFMANN-LA ROCHE & CO.)<br>* Patentansprüche; Seite 5, Zeile 1 - Seite 8, Zeile 20 *<br>----- | 1-5,8 | |

RECHERCHIERTE SACHGEBIETE (Int. Cl.5)

A 01 N
C 07 D

Der vorliegende Recherchenbericht wurde für alle Patentansprüche erstellt

| Recherchenort | Abschlußdatum der Recherche | Prüfer |
|---|---|---|
| DEN HAAG | 21-05-1991 | DONOVAN T.M. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)